(19) Europäisches Patentamt
European Patent Office
Office européen des brevets

(11) **EP 4 643 943 A1**

(12) **EUROPEAN PATENT APPLICATION**
published in accordance with Art. 153(4) EPC

(43) Date of publication:
**05.11.2025 Bulletin 2025/45**

(21) Application number: **22970265.9**

(22) Date of filing: **29.12.2022**

(51) International Patent Classification (IPC):
**A61N 7/00** *(2006.01)*      **A61B 5/0531** *(2021.01)*

(52) Cooperative Patent Classification (CPC):
**A61B 5/0531; A61N 7/00**

(86) International application number:
**PCT/KR2022/021656**

(87) International publication number:
**WO 2024/143627 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Healux Co., Ltd.**
**Seoul 08584 (KR)**

(72) Inventor: **KIM, Do Yeon**
**Incheon 22624 (KR)**

(74) Representative: **Jung, Minkyu**
**Wuesthoff & Wuesthoff**
**Patentanwälte und Rechtsanwalt PartG mbB**
**Schweigerstraße 2**
**81541 München (DE)**

(54) **SKIN TREATMENT DEVICE AUTOMATICALLY CONTROLLED ACCORDING TO CONDITION OF SKIN, AND OPERATION METHOD FOR SKIN TREATMENT DEVICE**

(57)    The present disclosure relates to a skin treatment device, the skin treatment device comprising: a main body including a control unit that controls the operation of the skin treatment device and a power source unit that supplies power to the medical skin treatment device; a transfer unit for moving the main body; and a handpiece which receives power from the power source unit of the main body, is controlled by the control unit, and has one side that comes into contact with the skin of a patient to radiate therapeutic energy below the skin surface of the patient and thus improve the skin.

Fig. 1

EP 4 643 943 A1

## Description

[Technical Field]

The present disclosure relates to a skin treatment device that is automatically controlled according to a condition of skin. In addition, the present disclosure relates to a skin treatment device having an improved type of transducer and a skin treatment device using an electrode. In addition, the present disclosure relates to a skin treatment device for injecting a drug.

[Background Art]

Cosmetic techniques for removing subcutaneous fat are increasingly being used in the field of medical skin treatment devices. Among the techniques for removing subcutaneous fat, there is a technique using ultrasound, and such an ultrasound technique is specifically classified into a planar ultrasound technique and a focused ultrasound technique. Among them, the fat removal effect and use safety of the focused ultrasound technique are higher than those of the planar ultrasound technique. The operation principle of the focused ultrasound technique is to install an ultrasound energy converter on a skin surface of a fat removal target area, intensively transmit ultrasound energy to the subcutaneous fat tissue, and rupture a fat cell membrane of a concentrated area through a cavity generated from high-energy ultrasound vibration so that contents of fat cells flow out and are removed through the body's own metabolism. Through such a process, the fat tissue in the fat removal target area is melted and removed, thereby achieving the purpose of fat removal and at the same time, not causing damage to surrounding normal tissues.

[Related Art]

Korean Laid-Open Patent No. 2016-0000143

[Technical Problem]

The present disclosure relates to a mobile skin treatment device, and the skin treatment device can improve skin. The mobile skin treatment device of the present disclosure can be miniaturized and moved freely. In addition, the present disclosure may provide different treatment methods depending on a condition of skin.

However, objects are not limited to the above objects, and other objects can be present.

[Technical Solution]

A medical skin treatment device according to the present disclosure includes a main body including a control unit configured to control an operation of the skin treatment device and a power source unit configured to supply power to the medical skin treatment device, a transfer unit configured to move the main body, and a handpiece that receives power from the power source unit of the main body, is controlled by the control unit, and has one side that comes into contact with a patient's skin to form a lesion based on vibration energy below a skin surface of the patient so as to improve skin, wherein the handpiece includes a base unit coupled to the one side of the handpiece, a plurality of transducers that are formed on one side of the base unit at positions excluding a center of the base unit and vibrate at a predetermined therapeutic frequency to transmit the vibration energy to the patient's skin, a thermoelectric element that is formed inside at least one of the base unit and the plurality of transducers and cools the at least one of the base unit and the plurality of transducers to prevent burning of the skin surface of the patient in contact with the one side of the handpiece, and an electrode unit that is formed on the one side of the base unit, includes at least one positive electrode and at least one negative electrode, and measures an impedance of the patient's skin, and the control unit determines an intensity of the vibration energy corresponding to the impedance of the patient's skin based on a predetermined function and emits the vibration energy from the plurality of transducers based on the intensity of the vibration energy.

In a coordinate system in which a horizontal axis represents the impedance and a vertical axis represents the intensity of the vibration energy, the predetermined function of the medical skin treatment device according to the present disclosure may be represented by a logarithmic function.

The control unit of the medical skin treatment device according to the present disclosure may cause the electrode unit to flow a predetermined test current from the at least one positive electrode to the at least one negative electrode, measure a test voltage between the at least one positive electrode and the at least one negative electrode, measure the impedance of the patient's skin based on the test current and the test voltage, determine the intensity of the vibration energy to be increased as the impedance increases based on the predetermined function, and control at least one of the plurality of transducers to emit the vibration energy based on the intensity of the vibration energy.

The control unit of the medical skin treatment device according to the present disclosure may control the electrode unit to apply a test voltage between the at least one positive electrode and the at least one negative electrode, measure a test current flowing between the at least one positive electrode and the at least one negative electrode, measure the impedance of the patient's skin based on the test current and the test voltage, determine the intensity of the vibration energy to be increased as the impedance increases based on the predetermined function, and control at least one of the plurality of transducers to emit the vibration energy based on the intensity of the vibration energy.

The predetermined therapeutic frequency of the vibration energy of the medical skin treatment device according to the present disclosure may be 250 kHz or more and 20 MHz or less.

A suction hole for suctioning the patient's skin may be formed in the base unit of the medical skin treatment device according to the present disclosure, and a negative pressure unit included in at least one of the handpiece or the main body may form a lower pressure than atmospheric pressure in the suction hole to suction the patient's skin.

A distance between the one side of the handpiece and the patient's bone may be measured based on the plurality of transducers included in the handpiece of the medical skin treatment device according to the present disclosure, when the measured distance is included in a threshold range and the pressure formed in the suction hole is less than the threshold pressure, the control unit may control the plurality of transducers to emit the vibration energy, and when the measured distance is not included in the threshold range or the pressure formed in the suction hole is higher than or equal to the threshold pressure, the control unit may control the plurality of transducers not to emit the vibration energy.

At least one of the threshold range and the threshold pressure may be determined based on the measured impedance of the medical skin treatment device according to the present disclosure.

A medical skin treatment device according to the present disclosure includes a main body including a control unit configured to control an operation of a skin treatment device and a power source unit configured to supply power to the medical skin treatment device, a transfer unit configured to move the main body, and a handpiece that receives power from the power source unit of the main body, is controlled by the control unit, and has one side that comes into contact with a patient's skin to form a lesion based on vibration energy below a skin surface of the patient so as to improve skin, wherein the handpiece includes a base unit that is coupled to one side of the handpiece, has a circular shape, and is rotatable with a center of a circle as a rotational axis, a plurality of transducers that are formed on the one side of the base unit, are rotatable together with the base unit, and vibrate at a predetermined therapeutic frequency to transmit vibration energy to the patient's skin, and a thermoelectric element that is formed inside at least one of the base unit and the plurality of transducers and cools the at least one of the base unit and the plurality of transducers to prevent burning of the skin surface of the patient in contact with the one side of the handpiece, and the control unit determines an intensity of the vibration energy and emits vibration energy from the plurality of transducers based on the intensity of the vibration energy.

The plurality of transducers of the medical skin treatment device according to the present disclosure may have different predetermined therapeutic frequencies.

The base unit of the medical skin treatment device according to the present disclosure may have a concave surface, and the plurality of transducers may be formed on the concave surface of the base unit.

The base unit of the medical skin treatment device according to the present disclosure may have a flat surface, the plurality of transducers may be formed on the flat surface of the base unit, and the plurality of transducers may have one of the concave surface and the flat surface.

The plurality of transducers of the medical skin treatment device according to the present disclosure may include a first transducer, a second transducer, and a third transducer, the predetermined therapeutic frequency of the first transducer may be 1 MHz, the predetermined therapeutic frequency of the second transducer may be 3 MHz, and the predetermined therapeutic frequency of the third transducer may be 10 MHz.

The first angle and the second angle of the medical skin treatment device according to the present disclosure may be the same, the first angle may be an angle formed by a first line connecting a center of the first transducer and a center of the base unit and a second line connecting a center of the second transducer and the center of the base unit, and the second angle may be an angle formed by a first line connecting the center of the first transducer and the center of the base unit and a third line connecting a center of the third transducer and the center of the base unit.

The control unit of the medical skin treatment device according to the present disclosure may control the base unit to rotate based on a user's input and emit the vibration energy, and a speed at which the base unit rotates may be determined based on an impedance of the patient's skin.

A suction hole for suctioning the patient's skin may be formed adjacent to the center of the circle of the base unit of the medical skin treatment device according to the present disclosure, a negative pressure unit included in at least one of the handpiece or the main body may form a lower pressure than atmospheric pressure in the suction hole to suction the patient's skin, a distance between the one side of the handpiece and the patient's bone may be measured based on the plurality of transducers included in the handpiece, when the measured distance is included in a threshold range and the pressure formed in the suction hole is less than the threshold pressure, the control unit may control the plurality of transducers to emit the vibration energy, and when the measured distance is not included in the threshold range or the pressure formed in the suction hole is higher than or equal to the threshold pressure, the control unit may control the plurality of transducers not to emit the vibration energy.

A medical skin treatment device according to the present disclosure includes a main body including a control unit configured to control an operation of a skin treatment device and a power source unit configured to supply power to the medical skin treatment device, a transfer unit configured to move the main body, and a handpiece that receives power from the power source unit of the main body, is controlled by the control unit, and has one side that comes into contact with a patient's skin to form a lesion based on vibration energy below a skin surface of the patient so as to improve skin, wherein the handpiece includes a base unit coupled to one side of the handpiece, at least one positive electrode that is formed on one side of the base unit and provides electrical energy to the patient's skin to improve the skin, and a thermoelectric element that is formed inside at least one of the base unit and the plurality of transducers and cools the at least one of the base unit and the plurality of transducers to prevent burning of the skin surface of the patient in contact with the one side of the handpiece, the control unit determines an intensity of the electrical energy corresponding to an impedance of the patient's skin based on a predetermined function and controls a therapeutic current to flow from the at least one positive electrode to the at least one negative electrode through the skin based on the intensity of the electrical energy and a predetermined therapeutic frequency, and the at least one negative electrode is formed on the one side of the base unit included in the handpiece or formed on a negative electrode pad independent of the handpiece.

A depth to which the electrical energy penetrates below the patient's skin surface via the handpiece of the medical skin treatment device according to the present disclosure may be inversely proportional to an electrode distance between the at least one positive electrode and the at least one negative electrode and inversely proportional to the therapeutic frequency.

The control unit of the medical skin treatment device according to the present disclosure may control a predetermined test current to flow from the at least one positive electrode to the at least one negative electrode, measure a test voltage between the at least one positive electrode and the at least one negative electrode, measure the impedance of the patient's skin based on the test current and the test voltage, determine the intensity of the electrical energy to be increased as the impedance increases based on the predetermined function, and emit electrical energy from the at least one positive electrode to the at least one negative electrode based on the intensity of the electrical energy.

The control unit of the medical skin treatment device according to the present disclosure may control a predetermined test current to be applied between the at least one positive electrode to the at least one negative electrode, measure a test current flowing between the at least one positive electrode and the at least one negative electrode, measure the impedance of the patient's skin based on the test current and the test voltage, determines the intensity of the electrical energy to be increased as the impedance increases based on the predetermined function, and emit electrical energy from the at least one positive electrode to the at least one negative electrode based on the intensity of the electrical energy.

In a coordinate system in which a horizontal axis represents impedance and a vertical axis represents the intensity of the electrical energy, the predetermined function of the medical skin treatment device according to the present disclosure may be represented by a logarithmic function.

The therapeutic frequency of the medical skin treatment device according to the present disclosure may be 250 KHz or more and 20 MHz or less.

A suction hole for suctioning the patient's skin may be formed in the base unit of the medical skin treatment device according to the present disclosure, a negative pressure unit included in at least one of the handpiece or the main body may form a lower pressure than atmospheric pressure in the suction hole to suction the patient's skin, and a level of the pressure formed in the suction hole may be determined based on the measured impedance.

A distance between the one side of the handpiece and the patient's bone may be measured based on a sensor included in the handpiece of the medical skin treatment device according to the present disclosure, and when the measured distance is within a threshold range and the pressure formed in the hole is lower than or equal to a threshold pressure, the control unit may control the base unit to emit electrical energy of a predetermined therapeutic frequency to the patient's skin.

A medical skin treatment device according to the present disclosure includes a main body including a control unit configured to control an operation of a skin treatment device and a power source unit configured to supply power to the medical skin treatment device, a transfer unit configured to move the main body, and a handpiece that receives power from the power source unit of the main body, is controlled by the control unit, and injects a drug into the patient's skin to improve the skin, wherein the handpiece includes a base unit formed on one side of the handpiece, and a drug injection unit that is formed on one side of the base unit and injects the drug into the patient's skin, and the drug injection unit includes a drug cylinder including a first chamber that receives and stores the drug from a drug supply unit and a second chamber that accommodates an expansion fluid for discharging the drug in the first chamber, a separator formed between the first chamber and the second chamber to prevent the fluid from flowing between the first chamber and the second chamber and formed of an elastic material, an injection valve formed in the second chamber to inject the expansion fluid of a predetermined mass into the second chamber, an ignition plug that is formed in the second chamber and expands the expansion fluid injected into the interior of the second chamber, and a discharge valve that is formed in the second chamber and discharges the expansion fluid expanded by the ignition plug from the second chamber.

The control unit of the medical skin treatment device according to the present disclosure may supply the drug from the drug supply unit to the first chamber, open the injection valve to inject the expansion fluid into the second chamber, close the injection valve and expand the expansion fluid using the ignition plug, and open the discharge valve to discharge the expanded expansion fluid from the second chamber through the discharge valve.

The first chamber of the medical skin treatment device according to the present disclosure may include a drug discharge port configured to discharge the drug and a drug injection port configured to receive the drug from the drug supply unit on a lower side thereof, the separator may be convex toward the first chamber by the expansion of the expansion fluid, and the separator may apply pressure to the drug inside the first chamber to discharge the drug through the drug discharge port.

The expanded expansion fluid may be discharged from the second chamber through the discharge valve by an elastic force of the separator of the medical skin treatment device according to the present disclosure.

The medical skin treatment device according to the present disclosure may include a pressure sensor configured to measure a pressure of the first chamber, wherein, to supply the drug from the drug supply unit to the first chamber, the control unit may stop the supply of the drug to the first chamber when the pressure of the first chamber is higher than or equal to a threshold pressure.

The control unit of the medical skin treatment device according to the present disclosure may open the discharge valve so that the expanded expansion fluid is discharged from the second chamber through the discharge valve and at the same time, supply the drug to the first chamber.

The first chamber of the medical skin treatment device according to the present disclosure may have a shape in which an inner diameter decreases downward or is constant, and the drug discharge port formed on a lower end of the first chamber may have an inner diameter of 10 micrometers or more and 300 micrometers or less.

The base unit of the medical skin treatment device according to the present disclosure may include at least one of a plurality of transducers that vibrate at a predetermined therapeutic frequency to transmit vibration energy to the patient's skin and at least one positive electrode configured to provide electrical energy to the patient's skin to improve the skin, and the drug injection port may be formed inside the plurality of transducers and at least one positive electrode.

In addition, a program for implementing an operation method of the above skin treatment device may be recorded on a computer-readable recording medium.

[Advantageous Effects]

A skin treatment device of the present disclosure includes various types of base units for the convenience of a user. The user can change the role of the skin treatment device by replacing the base unit. In addition, the base unit can include various functions, thereby reducing the user's burden of replacing the base unit.

Effects obtainable from the present disclosure are not limited to the above-described effects, and other effects that are not described will be clearly understood by those skilled in the art to which the present disclosure pertains based on the following description.

[Description of Drawings]

FIG. 1 is a view showing a medical skin treatment device according to one embodiment of the present disclosure.

FIG. 2 is a block diagram showing various components that may be included in the skin treatment device according to one embodiment of the present disclosure.

FIG. 3 is a side cross-sectional view showing a handpiece according to one embodiment of the present disclosure.

FIG. 4 is a view showing one side of a handpiece according to one embodiment of the present disclosure.

FIG. 5 is a view showing one side of the handpiece according to one embodiment of the present disclosure.

FIG. 6 is a flowchart for describing operations of the skin treatment device according to one embodiment of the present disclosure.

FIG. 7 is a flowchart for describing operations of the skin treatment device according to one embodiment of the present disclosure.

FIG. 8 is a view for describing a predetermined function according to one embodiment of the present disclosure.

FIG. 9 is a flowchart for describing operations of the skin treatment device according to one embodiment of the present disclosure.

FIG. 10 is a view showing one side of the handpiece according to one embodiment of the present disclosure.

FIG. 11 is a view showing a cross section of a base unit according to one embodiment of the present disclosure.

FIG. 12 is a view showing at least one positive electrode and at least one negative electrode according to one embodiment of the present disclosure.

FIG. 13 is a view for describing the handpiece according to one embodiment of the present disclosure.

FIG. 14 is a view showing the handpiece according to one embodiment of the present disclosure.

FIG. 15 is a view showing one side of the handpiece according to one embodiment of the present disclosure.

FIG. 16 is a view showing a cross section of a drug injection unit according to one embodiment of the present disclosure.

FIG. 17 is a flowchart showing operations of the skin treatment device according to one embodiment of the present disclosure.

[Modes of the Invention]

Advantages and features of the disclosed embodiments and methods for achieving them will become clear by referencing embodiments which will be described together with the accompanying drawings. However, the present disclosure is not limited to the embodiments to be disclosed below but can be implemented in various different forms, and these embodiments are merely provided to make the disclosure of the present disclosure complete and fully inform those skilled in the art to which the present disclosure pertains in the scope of the present disclosure.

Terms used in the present specification will be briefly described, and disclosed embodiments will be described in detail.

The terms used in the present specification are general terms that are currently widely used as much as possible while considering a function in the present disclosure, but this can vary depending on the intention or cases of a technician who works in the art, the emergence of a new technology, etc. In addition, in specific cases, there are terms arbitrarily selected by the applicant, and in this case, the meaning will be described in detail in the description of the corresponding invention. Accordingly, the terms used in the present disclosure should be defined based on the meanings of the terms and the overall content of the present disclosure rather than simply the names of the terms.

A singular expression used herein includes a plural expression unless the context clearly dictates otherwise. In addition, the plural expression includes the singular expression unless the context clearly dictates otherwise.

Throughout the specification, when a certain portion is described as "including" a certain component, it means further including another component rather than precluding another component unless especially stated otherwise.

In addition, the term "unit" used in the specification means a software or hardware component, and the "unit" performs certain roles. However, the "unit" is not limited to software or hardware. The "unit" may be configured in an addressable storage medium or configured to reproduce one or more processors. Accordingly, as an example, the "unit" includes components such as software components, object-oriented software components, class components, and task components, processes, functions, attributes, procedures, subroutines, segments of program code, drivers, firmware, microcode, circuits, data, database, data structures, tables, arrays, and variables. Functions provided in the components and "units" may be combined with a smaller number of components and "units" or separated into additional components and "units."

According to one embodiment of the present disclosure, the "unit" may be implemented as a processor and a memory. The term "processor" should be construed broadly to include general-purpose processors, central processing units (CPUs), microprocessors, digital signal processors (DSPs), controllers, microcontrollers, state machines, etc. In some circumstances, the "processor" may also refer to application-specific integrated circuits (ASICs), programmable logic devices (PLDs), field programmable gate arrays (FPGAs), etc. For example, the term "processor" may also refer to a combination of processing devices, such as a combination of a DSP and a microprocessor, a combination of multiple microprocessors, a combination of one or more microprocessors coupled with a DSP core, or any other such combination of components.

The term "memory" should be construed broadly to include any electronic component capable of storing electronic information. The term "memory" may refer to various types of processor-readable media, such as a random access memory (RAM), a read-only memory (ROM), a non-volatile RAM (NVRAM), a programmable ROM (PROM), an erasable programmable ROM (EPROM), an electrically erasable PROM (EEPROM), a flash memory, a magnetic or optical data storage, registers, etc. The memory is considered to function in an electronic communication state with the processor when the processor may read information from and/or write information on the memory. The memory integrated in the processor is in electronic communication with the processor.

In the present specification, an actuator means a component capable of providing a driving force. For example, the actuator may include a motor, a linear motor, an electric motor, a DC motor, an AC motor, a linear actuator, an electric actuator, etc., but is not limited thereto.

In the present disclosure, a lower direction (downward direction) may be a direction from a skin treatment device 100 to the ground, and an upper direction (upward direction) may be a direction from the ground to the skin treatment device 100.

Hereinafter, the present disclosure will be described in detail with reference to the accompanying drawings so that those skilled in the art to which the present disclosure pertains can easily carry out embodiments. In addition, to clearly describe the present disclosure in the drawings, parts that are not related to the description will be omitted.

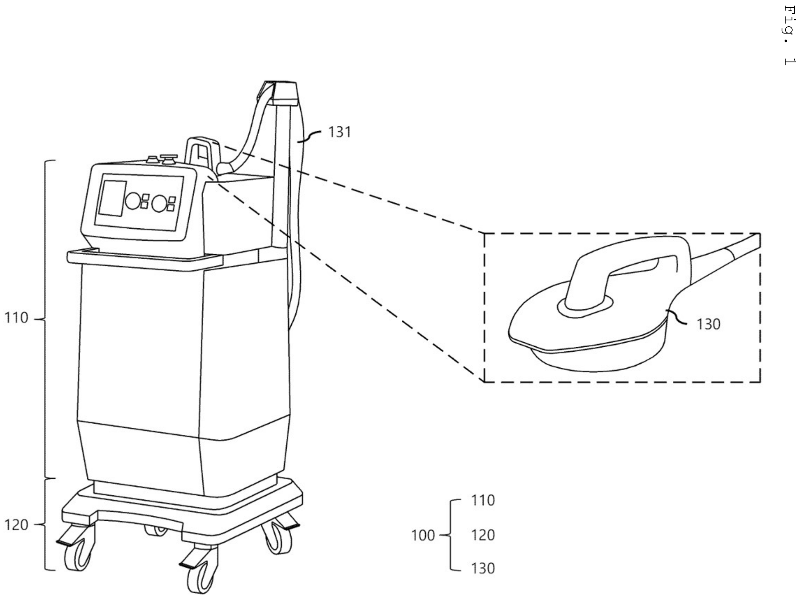

FIG. 1 is a view showing a medical skin treatment device according to one embodiment of the present disclosure.

The medical skin treatment device 100 may include a main body 110 including a control unit 200 and a power source unit. The main body 110 may further include at least one of a communication unit, a sensor unit, an output unit, and an input unit.

In addition, the medical skin treatment device 100 may include a transfer unit 120 for moving the main body 110. The transfer unit 120 may be located below the main body 110. The transfer unit 120 may include at least one wheel. The transfer unit 120 may include at least one of a caster wheel, an electric wheel, and a Mecanum wheel. The transfer unit 120 may include an electric motor, and the electric motor may provide a driving force based on a user's input. In addition, the transfer unit 120 may autonomously travel based on the control of the control unit 200. However, the present disclosure is not limited thereto, and the transfer unit 120 may be moved by the user's force.

In addition, the medical skin treatment device 100 may include a handpiece 130. The main body 110 may be connected to the handpiece 130 via a cable 131. The handpiece 130 may be used to treat a patient's skin. The handpiece 130 may include a handle that the user may hold. A surface that comes into contact with the patient's skin may be formed at one side of the handpiece 130. The main body 110 may supply power to the handpiece 130 via the cable 131 and transmit or receive a control signal in a wired manner.

Hereinafter, each function of the skin treatment device 100 will be described.

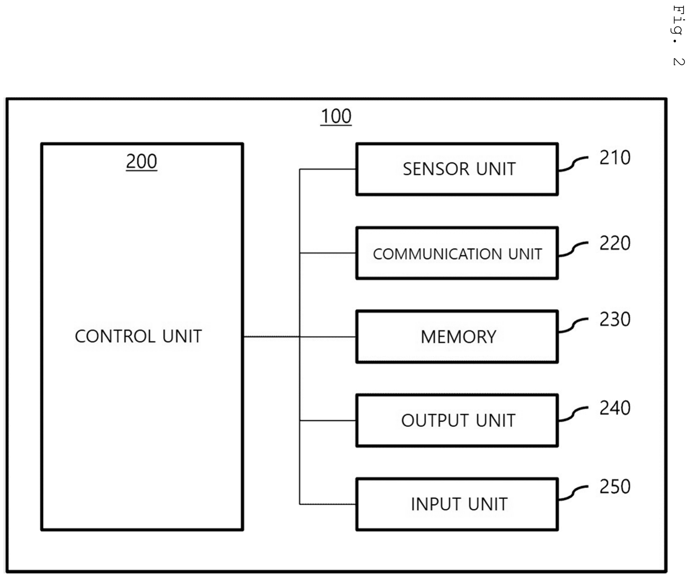

FIG. 2 is a block diagram showing various components that may be included in the skin treatment device according to one embodiment of the present disclosure.

The skin treatment device 100 may include a sensor unit 210, a communication unit 220, a memory 230, an output unit 240, an input unit 250, and the control unit 200.

The skin treatment device 100 may include the control unit 200. The control unit 200 may control the operation of the skin treatment device 100. For example, the skin treatment device 100 may include the control unit 200 for controlling the operation of at least one of a wheel and a handpiece that may drive the main body 110. The control unit 200 may include one processor or include a plurality of processors. The control unit 200 may be included in the main body 110. When the control unit 200 includes the plurality of processors, at least some of the plurality of processors may be provided at locations physically separated from the main body 110. In addition, the skin treatment device 100 is not limited thereto and may be implemented in various ways.

According to one embodiment of the present disclosure, the control unit 200 may control the operation of the skin treatment device 100. For example, the skin treatment device 100 may include a plurality of actuators, and the skin treatment device 100 may control the operation of the skin treatment device 100 by controlling the operation of the plurality of actuators. For example, the control unit 200 may control the operation of the handpiece 130 to perform an operation for improving the patient's skin.

The skin treatment device 100 may include the sensor unit 210. The sensor unit 210 may acquire various pieces of information using at least one sensor. The sensor unit 210 may be provided as a sensor that uses a measuring means such as pressure, an electric potential, optical characteristics, etc. For example, the sensor unit 210 may include at least one of a distance measuring sensor or an encoder. In addition, the sensor may include a pressure sensor, an infrared sensor, a light-emitting diode (LED) sensor, a touch sensor, etc. However, the present disclosure is not limited thereto. The sensor unit may be included in at least one of the main body 110, the transfer unit 120, and the handpiece.

In addition, the skin treatment device 100 may include the communication unit 220. The communication unit 220 may be a component through which the skin treatment device 100 communicates with an internal module or an external device in a wireless or wired manner. The external device may include an external server or a user terminal. The user terminal may include a PC, a smartphone, a tablet, or a wearable device. The communication unit 220 may include a wired/wireless communication module for network access. As wireless communication technologies, for example, wireless LAN (WLAN), Wi-Fi, wireless broadband (WiBro), world interoperability for microwave access (WiMAX), high speed downlink packet access (HSDPA), etc., may be used. For example, as wired communication technologies, a digital subscriber line (XDSL), fibers to the home (FTTH), power line communication (PLC), etc., may be used. In addition, a network connection unit may include a short-range communication module to transmit and receive data with any device/terminal located in a short range. For example, as short-range communication technologies, Bluetooth, radio frequency identification (RFID), infrared communication (infrared data association (IrDA)), ultra-wideband (UWB), ZigBee, etc., may be used, but the present disclosure is not limited thereto.

The skin treatment device 100 may include the memory 230. The control unit 200 may perform commands stored in the memory 230. The memory 230 may be included in the control unit 200 or located outside the control unit 200. The memory 230 may store various pieces of information related to the skin treatment device 100. For example, the memory 230 may include information related to the operation method of the handpiece 130 and include captured images and user authentication information, but is not limited thereto.

The memory 230 may be implemented through a non-volatile storage medium capable of maintaining stored arbitrary data. For example, the memory 230 may include not only a storage device based on a disk, an optical disk, and a magneto-optical storage device, but also a flash memory and/or a battery-backed memory, but is not limited thereto. The memory 230 may be a volatile storage device, such as a RAM such as a dynamic RAM (DRAM), a static RAM (SRAM), etc., which is a main storage device directly accessed by a processor and in which stored information is instantly erased when power is turned off, but is not limited thereto. The memory 230 may be operated by the control unit 200.

In addition, the skin treatment device 100 may further include a manipulation unit for providing an interface for manipulating the skin treatment device 100. The manipulation unit may include the output unit 240 and the input unit 250.

The output unit 240 may output information related to treatment, information related to a patient, information related to the control of a handpiece, and information related to a state of a main body as sounds and images under the control of the control unit 200. The output unit 240 may include a speaker or a display. The output unit 240 may output medical images generated by the control unit 200. The output unit 240 may output information necessary for a user to manipulate the skin treatment device 100, such as a user interface (UI), user information, target information, etc. Examples of the output unit 240 may include a speaker, a printer, a negative electrode ray tube (CRT) display, a liquid crystal display (LCD), a plasma display panel (PDP) display, an organic light-emitting diode (OLED) display, a field emission display (FED), an LED display, a vacuum fluorescent display (VFD), a digital light processing (DLP) display, a flat panel display (FPD), a 3D display, a transparent display, etc., and include various output devices within a range that is obvious to those skilled in the art.

The output unit 240 may display information related to treatment. The information related to treatment may include at least one of a treatment method to be provided to a patient, a treatment time, and information related to the patient's disease. The treatment method may include at least one of ultrasound treatment, radio frequency (RF) treatment, and drug injection. The output unit 240 may include information related to the patient. The information related to the patient may include at least one of the patient's age, sex, treatment period, and information related to the disease. The information related to the control of the handpiece may include at least one of a button for controlling the handpiece, an operation cycle, a type of base unit currently connected to the handpiece, the intensity of energy used for treatment, and a frequency. Information related to a state of a main body may include at least one of a state of a battery of the main body, a power connection state, and a state of an error.

In addition, the output unit 240 may output information related to the control of the handpiece 130. For example, the control unit 200 may control the output unit 240 to output sound or light so that nearby people may know the handpiece being operated when the handpiece 130 operates.

The skin treatment device 100 may be connected to a workstation in a wired or wireless manner. The workstation may be present in a space physically separated from the skin treatment device 100.

The workstation may include a storage server. The storage server may store at least one of information about the patient, information about the treatment, and information about the user (medical professional). The workstation may include a review device. The review device may receive a medical image from the storage server based on a user's command and display the medical image. The workstation and the skin treatment device 100 may transmit, store, process, and output data according to the digital imaging and communications in drug (DICOM) standard. In addition, the workstation may include a picture archiving and communication system (PACS).

The workstation may include an output unit, an input unit, and a control unit. The output unit and the input unit provide a user with a user interface for manipulating the workstation and the skin treatment device 100. The control unit of the workstation may control the workstation and the skin treatment device 100.

The skin treatment device 100 may be controlled through the workstation and may also be controlled by the control unit 200 included in the skin treatment device 100. Accordingly, the user may control the skin treatment device 100 through the workstation or control the skin treatment device 100 through the manipulation unit and the control unit 200 included in the skin treatment device 100. That is, the user may remotely control the skin treatment device 100 through the workstation or directly control the skin treatment device 100.

The control unit of the workstation and the control unit 200 of the skin treatment device 100 may be separate but are not limited thereto. The control unit of the workstation and the control unit 200 of the skin treatment device 100 may be implemented as a single integrated control unit, or the integrated control unit may be included in only one of the workstation and the skin treatment device 100. Hereinafter, the control unit 200 may be the control unit of the workstation and/or the control unit of the skin treatment device 100.

The output unit and input unit of the workstation and the output unit 240 and input unit 250 of the skin treatment device 100 may each provide the user with an interface for manipulating the skin treatment device 100. The workstation and the skin treatment device 100 may each include an output unit and an input unit but are not limited thereto. The output unit or the input unit may be implemented in only one of the workstation and the skin treatment device 100.

Hereinafter, the input unit 250 is the input unit of the workstation and/or the input unit of the skin treatment device 100, and the output unit 240 is the output unit of the workstation and/or the output unit of the skin treatment device 100.

The input unit 250 may receive commands for manipulating the skin treatment device 100 and various pieces of information regarding X-ray photography from the user. The control unit 200 may control or manipulate the skin treatment device 100 based on the information input to the input unit 250. The input unit 250 may include a joystick, a keyboard, a mouse, a touch screen, a shooting button, a locking release button, a voice recognition device, a fingerprint recognition device, an iris recognition device, etc. and include other input devices that are obvious to those skilled in the art. The user may input a command for operating the handpiece through the input unit 250, and the input unit 250 may be provided with a switch for inputting such a command.

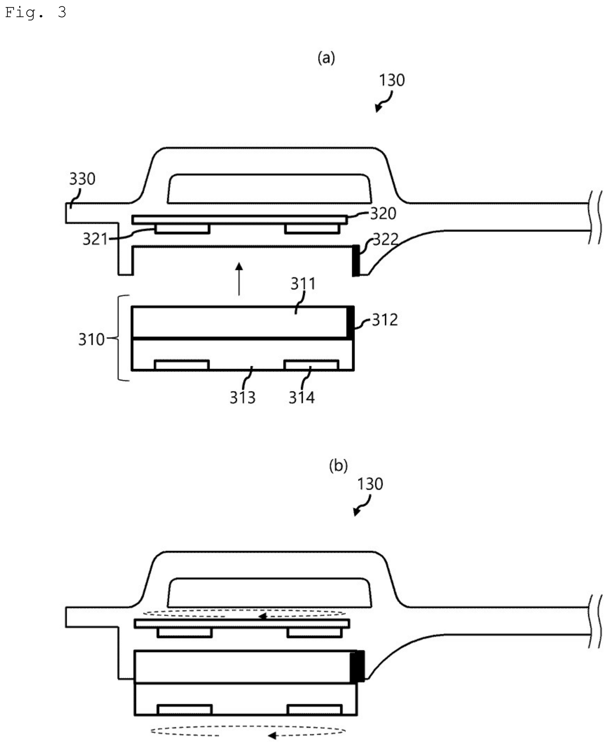

FIG. 3 is a side cross-sectional view showing a handpiece according to one embodiment of the present disclosure.

Referring to FIGS. 3A and 3B, the handpiece 130 may receive power from a power source unit of the main body 110. The handpiece 130 may be controlled by the control unit 200. One side of the handpiece 130 may come into contact with the patient's skin to form a lesion based on vibration energy under the skin surface of the patient, thereby improving the skin. Hereinafter, components included in the handpiece 130 will be described in more detail. For example, subcutaneous tissue may be destroyed by vibration energy, or the temperature of the tissue may be increased by vibration energy to form a lesion. The skin can be improved during the process of recovering the destroyed tissue of the patient. In addition, the destroyed tissue is adipose tissue, and since the adipose tissue is destroyed by the handpiece 130, it is possible to reduce the subcutaneous fat of the skin.

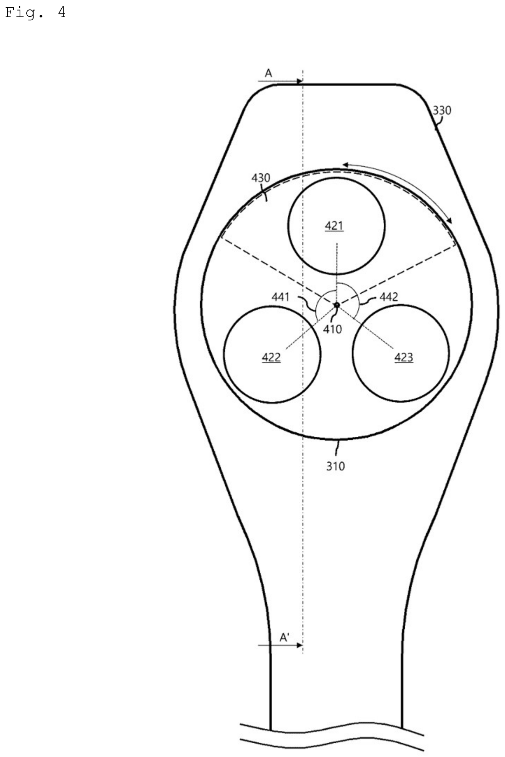

The handpiece 130 may include a base unit 310 coupled to the one side of the handpiece 130. The coupling may be implemented by hardware fastening. The coupling may use a general hardware fastening method. The base unit 310 may have one of a circle and a polygon. The circle-shaped base unit 310 is disclosed in FIG. 4, but the present disclosure is not limited thereto. Referring to FIG. 3A, the base unit 310 of the handpiece 130 may be detachably attached. A connector 312 may be formed on the base unit 310. In addition, a connector 322 may be formed on a main body 330 of the handpiece 130. Referring to FIG. 3B, when the base unit 310 is coupled to the handpiece 130, the connector may be connected. The base unit 310 may receive power from the handpiece 130 through the connector. In addition, the base unit 310 may transmit and receive signals with the handpiece 130 through the connector.

The skin treatment device 100 may be coupled to at least one of a plurality of base units 310 having different purposes. Based on the user's selection, the skin treatment device 100 may be coupled to one of the plurality of base units 310. When the handpiece 130 is coupled to the base unit 310, the handpiece 130 may acquire identification information of the base unit 310. More specifically, when the base unit 310 is coupled to the main body 330 of the handpiece 130, the main body 330 of the handpiece 130 may supply power to the base unit 310. In addition, the main body 330 of the handpiece 130 may transmit an identification information request signal to the base unit 310. The base unit 310 may transmit the identification information to the main body 330 of the handpiece 130 in response to a request from the handpiece 130 or by supplying power. In addition, the main body of the handpiece 130 may include an image capturing unit as the sensor unit 210. The handpiece 130 may read a code written on the base unit 310 using the image capturing unit to acquire the identification information of the base unit 310. In addition, the connector 322 of the handpiece 130 may include a plurality of pins. The plurality of base units 310 may have different connectors 312 according to the purpose. That is, one base unit 310 may be connected to some of the plurality of pins included in the connector 322 of the handpiece 130. The main body 330 of the handpiece 130 may determine the identification information of the base unit 310 based on the pin connected to the base unit 310 among the pins of the connectors 322.

FIG. 4 is a view showing one side of the handpiece according to one embodiment of the present disclosure.

A cross-section along line A-A' of FIG. 4 may be the same as FIG. 3. One side of the handpiece 130 may be a surface on which the handpiece 130 comes into contact with the user.

The base unit 310 may rotate with respect to the main body 330 of the handpiece 130. The base unit 310 may rotate about a rotational axis 410. The base unit 310 may have a circular shape.

The base unit 310 may include a fixed portion 311 and a rotating portion 313. The fixed portion of the base unit 310 may be a portion to be coupled to the main body 330 of the handpiece 130 and may be fixed. The connector 312 may be formed on the fixed portion 311. The rotating portion included in the base unit 310 may rotate with respect to the fixed portion 311. The rotating portion 313 may include at least one of a plurality of transducers, at least one positive electrode, and a drug injection unit. As described above, the handpiece 130 may be coupled to various types of base units 310, and the type of the base unit 310 may be determined by a combination of at least one of the plurality of transducers, the at least one positive electrode, and the drug injection unit.

The reference numeral 314 may denote at least one of the plurality of transducers, the at least one positive electrode, and the drug injection unit. The plurality of transducers, the at least one positive electrode, and the drug injection unit will be described below.

As described above, the skin treatment device 100 may be coupled to at least one of the plurality of base units 310 having different purposes. The plurality of base units 310 having different purposes may include at least one of the plurality of transducers, the at least one positive electrode, and the drug injection unit. For example, a first base unit may include a plurality of transducers, a second base unit can include at least one positive electrode, and a third base unit may include a drug injection unit. The user may couple one of the first to third base units to the main body 330 of the handpiece as needed. Hereinafter, the base unit 310 including at least one of a plurality of transducers, at least one positive electrode, and a drug injection unit will be described.

The base unit 310 may include the plurality of transducers. One side of the base unit 310 may include a plurality of transducers 421, 422, and 423 formed at positions excluding a center of the base unit 310. Here, the center of the base unit 310 may be the rotational axis 410. In addition, the one side of the base unit 310 may be a surface in contact with the patient's skin. The plurality of transducers 421, 422, and 423 may vibrate at a predetermined therapeutic frequency to transmit vibration energy to the patient's skin. Here, the predetermined therapeutic frequency of the vibration energy may be 250 kHz or more and 20 MHz or less.

The plurality of transducers 421, 422, and 423 may have a circular shape. In addition, a center of the plurality of transducers 421, 422, and 423 may be spaced a predetermined distance from the rotational axis 410. The sizes of the plurality of transducers may all be the same. However, the present disclosure is not limited thereto.

At least one of the base unit 310 and the plurality of transducers 421, 422, and 423 may include a thermoelectric element (not shown). The thermoelectric element may be formed inside at least one of the base unit 310 and the plurality of transducers 421, 422, and 423. The thermoelectric element may cool at least one of the base unit and the plurality of transducers. Accordingly, the thermoelectric element can prevent burning of the skin surface of the patient in contact with the one side of the handpiece 130.

In the above description, the reference numerals 421, 422, and 423 are described as denoting the plurality of transducers, but are not limited thereto. The reference numerals 421, 422, and 423 may also be at least one of at least one positive electrode and the drug injection unit.

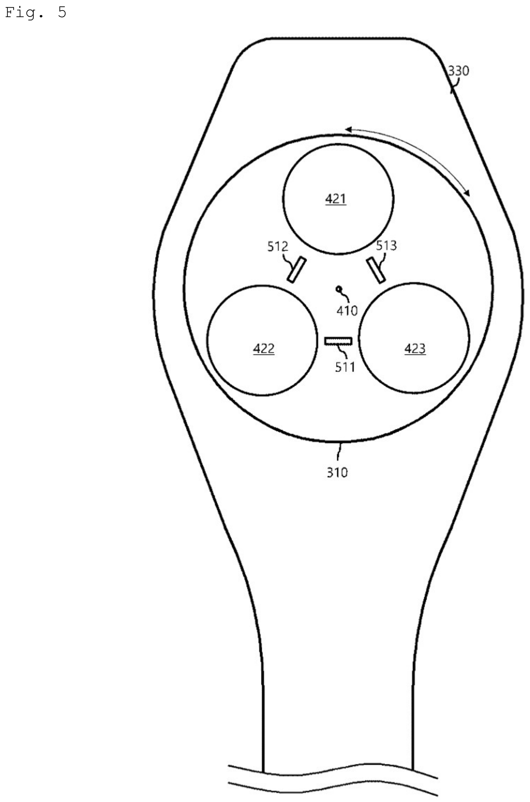

FIG. 5 is a view showing one side of the handpiece according to one embodiment of the present disclosure.

FIG. 5 is a view for further describing a component added in FIG. 4. The description of FIG. 5 that overlaps the description of FIG. 4 will be omitted.

The handpiece 130 may include an electrode unit 511, 512, and 513. The electrode unit 511, 512, and 513 may be formed on one side of the base unit 310 and may include at least one positive electrode and at least one negative electrode. The electrode unit 511, 512, and 513 may include two positive electrodes and one negative electrode. However, the present disclosure is not limited thereto, and the electrode unit 511, 512, and 513 may include two positive electrodes and one negative electrode. In addition, unlike FIG. 5, the electrode unit 511, 512, and 513 may include one positive electrode and one negative electrode. The electrode unit 511, 512, and 513 may be disposed between the plurality of transducers 421, 422, and 423.

The electrode may be formed on the rotational axis 410. More specifically, the plurality of positive electrodes 511, 512, and 513 may be formed on the one side of the base unit 310. In addition, a negative electrode may be formed on the rotational axis 410.

The control unit 200 may determine the intensity of vibration energy corresponding to an impedance of the patient's skin based on a pre-determined function and emit vibration energy from the plurality of transducers based on the intensity of the vibration energy.

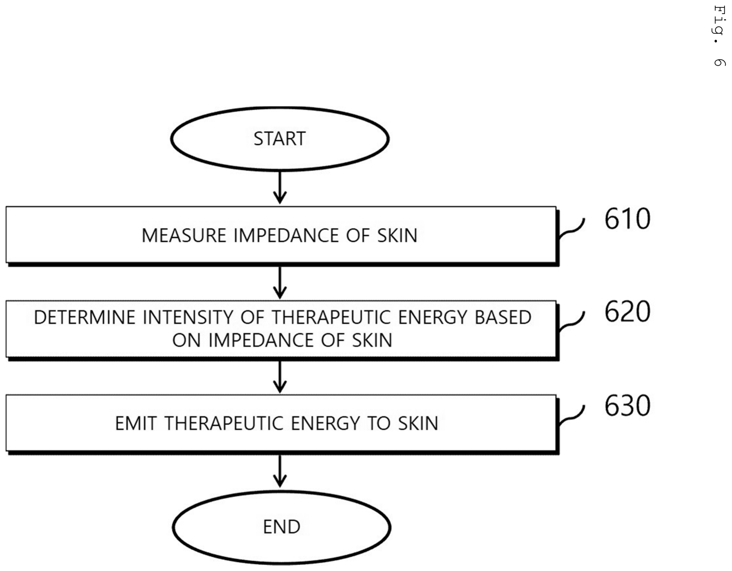

A process of the control unit 200 measuring the impedance of the patient's skin will be described with reference to FIGS. 6 to 8 below.

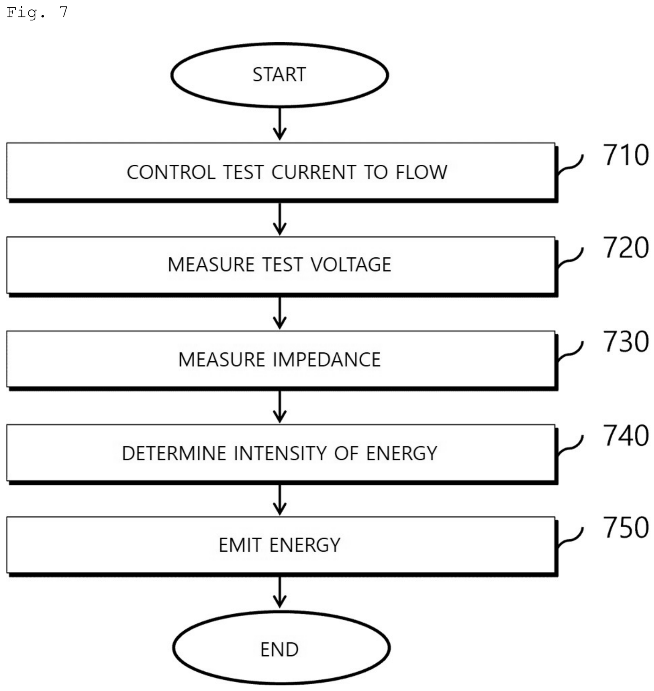

FIG. 6 is a flowchart for describing operations of the skin treatment device according to one embodiment of the present disclosure. FIG. 7 is a flowchart for describing operations of the skin treatment device according to one embodiment of the present disclosure.

FIGS. 6 and 7 will be described based on an example in which the plurality of transducers are provided in the base unit 310. However, the present disclosure is not limited thereto, and the same description may be applied even when at least one positive electrode and the drug injection unit are provided in the base unit 310.

Referring to FIG. 6, the control unit 200 may perform operation 610 of measuring the impedance of the patient's skin by allowing current to flow through the patient's skin or applying voltage to the patient's skin using the electrodes 511, 512, and 513. The control unit 200 may perform operation 620 of determining the intensity of therapeutic energy corresponding to the impedance of the patient's skin based on a pre-determined function. The control unit 200 may perform operation 630 of emitting therapeutic energy from the plurality of transducers based on the intensity of the vibration energy. Here, the therapeutic energy may include at least one of vibration energy and electrical energy. When the base unit 310 includes a transducer, the therapeutic energy will be vibration energy. In addition, when the base unit 310 includes at least one positive electrode, the therapeutic energy will be electrical energy.

The therapeutic energy including the vibration energy may be provided to the patient in the form of a therapeutic waveform. The intensity (size) of the therapeutic energy may be determined by a peak value of the therapeutic waveform. The therapeutic waveform may have a therapeutic frequency. The therapeutic frequency may be 250 kHz or more and 20 MHz or less. The therapeutic waveform may be a sine wave. The intensity (size) of the therapeutic energy may be determined by the peak value of the therapeutic waveform, which is a sine wave. The therapeutic waveform may be in the form of a pulse. That is, the therapeutic waveform may be a form in which sine waves are provided for a predetermined first time, and the waves are not provided for a predetermined second time after the first time has elapsed. The first time and the second time may be repeated in succession.

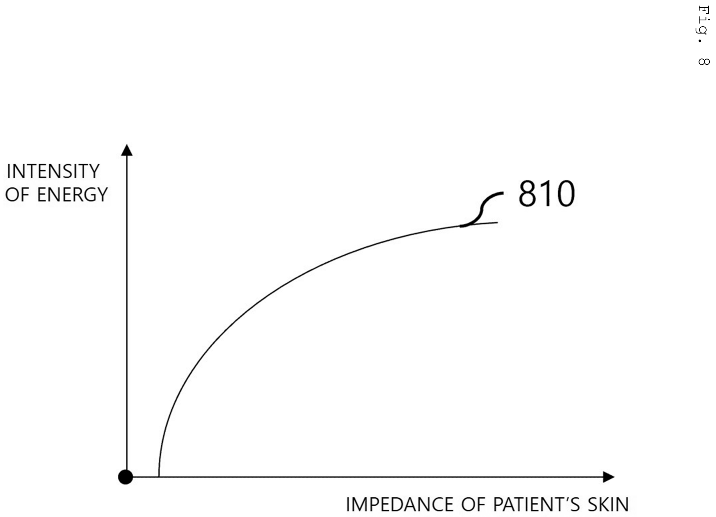

Each operation of FIG. 6 will be described in more detail with reference to FIGS. 7 and 8.

Referring to FIG. 7, the control unit 200 may perform operation 710 of controlling the electrodes 511, 512, and 513 so that a predetermined test current flows from at least one positive electrode to at least one negative electrode. The test current may flow from at least one positive electrode to at least one negative electrode through the patient's skin. The test current is a minute current, and the patient may not be aware that the current is flowing. The control unit 200 may stop the rotation of the base unit while performing operation 710.

The control unit 200 may perform operation 720 of measuring a test voltage between at least one positive electrode and at least one negative electrode. When the electrode unit includes a plurality of positive electrodes, the control unit 200 may sequentially flow the current to each positive electrode. The control unit 200 may measure a plurality of test voltages measured by flowing the current to each of the plurality of positive electrodes. The control unit 200 may determine that a minimum value of the plurality of test voltages or a median or average value of the plurality of test voltages is a representative test voltage.

As described above, the plurality of positive electrodes 511, 512, and 513 may be formed on one side of the base unit 310. In addition, a negative electrode may be formed on the rotational axis 410. The plurality of positive electrodes 511, 512, and 513 may come into firm contact with the patient's skin, but some electrodes may not be in contact with the patient's skin due to the uneven skin of the patient. When the electrodes may not be in contact with the patient's skin, the accurate impedance of the patient's skin cannot be obtained. The control unit 200 can accurately measure the impedance of the patient's skin using some of the plurality of test voltages or using the average or median value as the representative test voltage.

The control unit 200 may perform operation 730 of measuring the impedance of the patient's skin based on the test current and the test voltage. The control unit 200 may measure the impedance based on Ohm's law.

The control unit 200 may perform operation 740 of determining the intensity of the vibration energy to be increased as the impedance increases based on a predetermined function 810. That is, the impedance of the patient's skin may be directly proportional to the intensity of the vibration energy. However, the present disclosure is not limited thereto, and the impedance of the patient's skin may be inversely proportional to the intensity of the vibration energy. FIG. 8 is briefly referenced to describe the predetermined function. Here, the vibration energy may be a value proportional to the square of the amplitude of the wave.

FIG. 8 is a view for describing a predetermined function according to one embodiment of the present disclosure.

Referring to FIG. 8, the predetermined function 810 may be represented in a coordinate system in which a horizontal axis represents impedance and a vertical axis represents an intensity of vibration energy. As shown in FIG. 8, the predetermined function 810 may be represented as a logarithmic function. For example, the form of the logarithmic function may be as follows.

$$S = A * \log(x) + B$$

Here, A and B are predetermined constants and are real numbers. x denotes the impedance of the patient's skin. S denotes the intensity of the vibration energy. Since the predetermined function 810 has the logarithmic function, the intensity of the energy may vary greatly depending on the impedance within a small impedance range. However, within a large impedance range, the intensity of the energy may hardly vary depending on the impedance. Accordingly, it is possible to prevent excessively strong energy from being supplied to the patient. In addition, by varying the intensity of the energy depending on the impedance, the skin improvement effect can be maximized. In addition, low impedance may mean that the skin has increased moisture. On the contrary, high impedance may mean that the skin has decreased moisture. Generally, skin with increased moisture is known as healthy skin, and as aging progresses, the skin easily dries out. Improving the skin may require treatment with a high level of energy. The skin treatment device of the present disclosure may automatically determine therapeutic energy according to impedance, thereby increasing user convenience.

Referring back to FIG. 7, the control unit 200 may perform operation 750 of controlling at least one of the plurality of transducers to emit the vibration energy based on the intensity of the vibration energy.

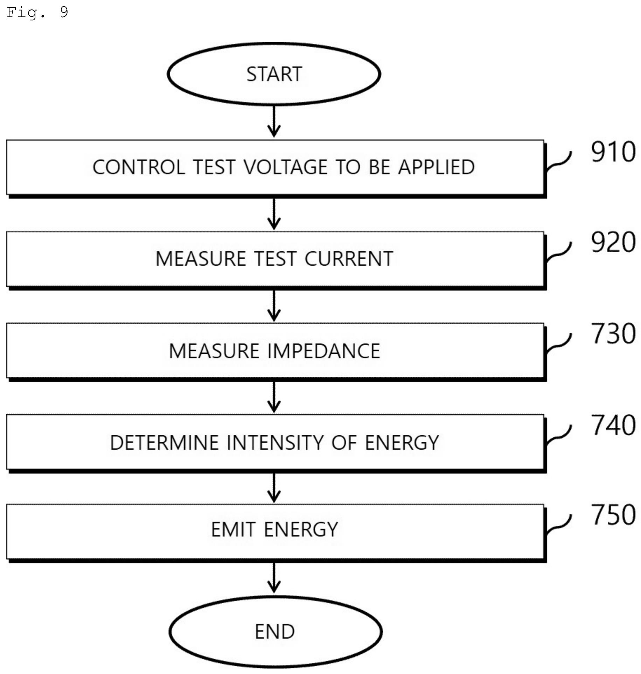

FIG. 9 is a flowchart for describing operations of the skin treatment device according to one embodiment of the present disclosure.

FIG. 9 may be a different embodiment from that of FIG. 7. When the test voltage is measured after the test current is applied in FIG. 7, the test current may be measured after the test voltage is applied in FIG. 9.

The control unit 200 may perform operation 910 of controlling the electrode unit to apply the test voltage between at least one positive electrode and at least one negative electrode. When the test voltage is applied to the at least one positive electrode and the at least one negative electrode, the test current may flow through the patient's skin. The test current may flow from at least one positive electrode to at least one negative electrode through the patient's skin. The test current is a minute current, and the patient may not be aware that the current is flowing. The control unit 200 may stop the rotation of the base unit while performing operation 910.

The control unit 200 may perform operation 920 of measuring a test current between the at least one positive electrode and the at least one negative electrode. When the electrode unit includes a plurality of positive electrodes, the control unit 200 may sequentially apply voltage to each positive electrode. The control unit 200 may measure a plurality of test currents by applying voltage to the plurality of positive electrodes. For example, a first test current may be measured by applying voltage between a first positive electrode and the negative electrode, and a second test current can be measured by applying voltage between a second positive electrode and the negative electrode. The control unit 200 may determine that the minimum value among the plurality of test currents or the median or average value of the plurality of test currents is the representative test current.

As described above, the plurality of positive electrodes 511, 512, and 513 may be formed on one side of the base unit 310. In addition, a negative electrode may be formed on the rotational axis 410. The plurality of positive electrodes 511, 512, and 513 may come into firm contact with the patient's skin, but some electrodes may not be in contact with the patient's skin due to the uneven skin of the patient. When the electrodes may not be in contact with the patient's skin, the accurate impedance of the patient's skin cannot be obtained. The control unit 200 can accurately measure the impedance of the patient's skin using some of the plurality of test currents or using the average or median value as the representative test current.

The control unit 200 may perform operation 730 of measuring the impedance of the patient's skin based on the test current and the test voltage. Operation 730 of FIG. 9 may correspond to operation 730 of FIG. 7.

The control unit 200 may perform operation 740 of determining a higher intensity of the vibration energy as the impedance increases based on the predetermined function. Operation 740 of FIG. 9 may correspond to operation 740 of FIG. 7.

The control unit 200 may perform operation 750 of controlling at least one of the plurality of transducers to emit the vibration energy based on the intensity of the vibration energy. Operation 750 of FIG. 9 may correspond to operation 750 of FIG. 7.

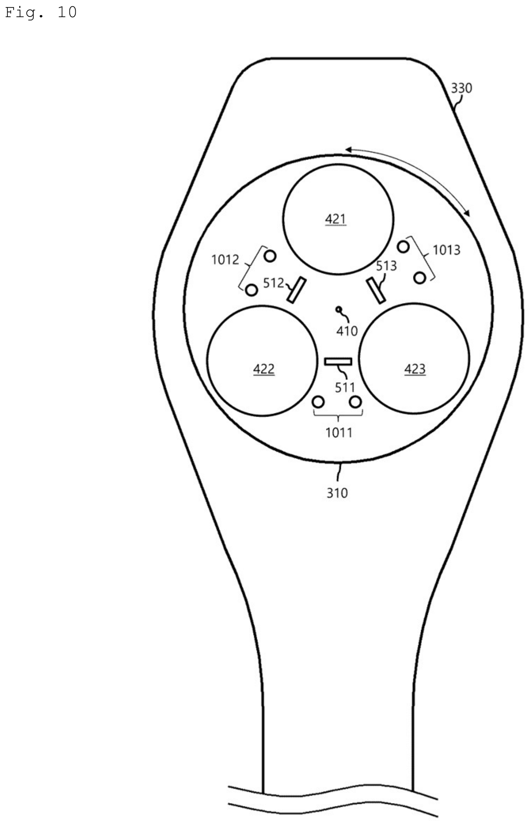

FIG. 10 is a view showing one side of the handpiece according to one embodiment of the present disclosure.

FIG. 10 is a view for further describing components added in FIGS. 4 and 5. The parts already described with reference to FIGS. 4 and 5 will be omitted in the description of FIG. 10.

The skin treatment device 100 may include suction holes 1011, 1012, and 1013. The suction holes 1011, 1012, and 1013 may be components that are formed in the base unit 310 and suction the patient's skin. At least one of the handpiece 130 or the main body 110 may include a negative pressure unit (not shown). The negative pressure unit may be a component configured to form a lower pressure than atmospheric pressure. For example, the negative pressure unit may be a compressor. When the negative pressure unit is formed in the main body 110, negative pressure may be transmitted to the suction holes 1011, 1012, and 1013 through a tube included in the cable 131. The handpiece 130 or the main body 110 may include a pressure sensor for measuring negative pressures formed in the suction holes 1011, 1012, and 1013.

The skin treatment device 100 may generate a lower pressure than the atmospheric pressure in the suction holes 1011, 1012, and 1013 by the negative pressure unit. The suction holes 1011, 1012, and 1013 may have a lower pressure than the atmospheric pressure to suction the patient's skin. Accordingly, the plurality of transducers 421, 422, and 423 formed in the base unit 310 may come into close contact with the patient's skin. The plurality of transducers 421, 422, and 423 may treat a predetermined location below the skin surface of the patient. To treat the predetermined location, the plurality of transducers 421, 422, and 423 need to be in contact with the skin. When the plurality of transducers 421, 422, and 423 are not in contact with the skin, the plurality of transducers 421, 422, and 423 may not transmit energy to the predetermined location. Based on the suction holes 1011, 1012, and 1013, the plurality of transducers 421, 422, and 423 come into close contact with the skin, and thus may always treat the predetermined location. Accordingly, the treatment precision of the skin treatment device can be increased.

The control unit 200 may measure a distance between the one side of the handpiece 130 and the patient's bone based on the plurality of transducers 421, 422, and 423 included in the handpiece 130. The distance between the one side of the handpiece 130 and the patient's bone may be a distance from the skin surface to the bone. The plurality of transducers 421, 422, and 423 may generate ultrasound toward the patient's skin. The plurality of transducers 421, 422, and 423 may also receive ultrasound reflected from tissues under the patient's skin. The control unit 200 may measure the distance from the skin surface to the bone based on the reflected ultrasound. The control unit 200 may measure the distance from the skin surface to the bone based on the plurality of transducers 421, 422, and 423 before emitting therapeutic energy to the skin.

When the measured distance between the one side of the handpiece 130 and the patient's bone is within a threshold range and the pressure formed in the suction hole is less than a threshold pressure, the control unit 200 may control the plurality of transducers to emit the vibration energy. Here, the threshold range may be a first threshold distance or more and a second threshold distance or less. The handpiece 130 or the main body 110 may include a pressure sensor for measuring negative pressures formed in the suction holes 1011, 1012, and 1013. Based on the pressure sensor, the control unit 200 may measure the pressure formed in the suction holes 1011, 1012, and 1013.

In addition, when the measured distance is not included in the threshold range or the pressure formed in the suction hole is higher than or equal to the threshold pressure, the control unit 200 may control the plurality of transducers not to emit the vibration energy. In this way, by controlling the plurality of transducers to emit the vibration energy only when the measured distance between the one side and the patient's bone is included in the threshold range and the pressure formed in the suction hole is less than the threshold pressure, the patient's safety can be secured, and accurate treatment can be performed. This is because the skin treatment device can perform accurate treatment only when the distance between the one side of the handpiece 130 and the patient's bone is included in the threshold range. When the distance between the one side of the handpiece 130 and the patient's bone is outside the threshold range, it may damage nerves or muscles below the skin, thereby causing harm to the patient. In addition, when the pressure formed in the suction hole is less than the threshold pressure, it means that the suction hole holds the patient's skin, which may mean that it is ready to safely emit the therapeutic energy to the patient.

In addition, each of the plurality of transducers 421, 422, and 423 may measure the distance between one side and the patient's bone. The control unit 200 may control only at least one of the plurality of transducers 421, 422, and 423, in which the measured distance between the one side and the patient's bone is within the threshold range, to emit the vibration energy. Controlling only the prepared transducers 421, 422, and 423 to emit the vibration energy may mean that it is ready to safely emit the therapeutic energy to the patient.

The control unit 200 may determine at least one of the threshold range and the threshold pressure based on the measured impedance. The control unit 200 may store a table or function in which the impedance matches with the threshold range. In addition, the control unit 200 may store a table or function in which the impedance matches with the threshold pressure. The control unit 200 may determine at least one of the threshold range and the threshold pressure based on at least one of the table and the function. The higher the measured impedance, the narrower the threshold range. In addition, the higher the measured impedance, the smaller the threshold pressure. However, the present disclosure is not limited thereto, and the higher the measured impedance, the wider the threshold range. In addition, the higher the measured impedance, the higher the critical pressure.

Hereinafter, a skin treatment device according to various embodiments of the present disclosure will be described. The parts that are already described above will be omitted.

Referring to FIGS. 4, 5, and 10, the handpiece 130 may include the base unit 310. The base unit 310 may be formed in a circular shape on one side of the handpiece. In addition, the base unit 310 may rotate about a center of the circle as the rotational axis 410. As described above, the base unit 310 may include the fixed portion and the rotating portion. A rotatable portion of the base unit 310 may be the rotating portion.

Referring to FIG. 3, the main body 330 of the handpiece 130 may include a configuration for rotating the base unit 310. More specifically, the main body 330 of the handpiece 130 may include a guide rail 320 and a driving coupling portion 321 that moves along the guide rail 320. For example, the driving coupling portion 321 may be a permanent magnet or an electromagnet. The driving coupling portion 321 may move along the guide rail 320. The guide rail 320 may have a ring shape. The driving coupling portion 321 may move along the guide rail 320 based on the actuator included in the main body 330 of the handpiece 130. The driving coupling portion 321 may rotate along the ring-shaped guide rail 320.

The driving coupling portion 321 may be magnetically coupled to a metal or magnet of the rotating portion 313 of the base unit 310. Accordingly, when the driving coupling portion 321 moves along the guide rail 320, the rotating portion of the base unit 310 may also move. In this way, since the rotating portion of the base unit 310 may be rotated by the magnetic coupling of the driving coupling portion 321 and the base unit 310, excessive stimulation may not be applied to the patient's skin. This is because, when the patient's skin blocks the rotation of the base unit 310, only the driving coupling portion 321 may rotate in a state in which the base unit 310 stops. When the patient's skin does not obstruct the rotation of the base unit 310, the base unit 310 may resume rotation along the driving coupling portion 321. However, the present disclosure is not limited thereto, and the rotating portion 313 of the base unit 310 may be rotated by the actuator.

The control unit 200 may control the base unit to rotate based on the user's input and emit vibration energy. The control unit 200 may determine at least one of the presence or absence of rotation, rotation speed, and rotation direction of the base unit 310 based on the user's input.

In addition, the rotation speed of the base unit 310 may be automatically determined based on the impedance of the patient's skin. More specifically, the control unit 200 may control the rotation speed of the base unit 310 to be slowed down as the impedance of the skin increases. The more moisture there is in the skin, the lower the impedance of the skin. In this way, the control unit 200 may slow down the rotation speed as the moisture content of the skin decreases, thereby allowing the vibration energy to be completely transmitted to the skin. However, the present disclosure is not limited thereto, and the rotation speed of the base unit 310 may be controlled to be fast as the impedance increases.

Referring to FIG. 4, the base unit 310 may include the plurality of transducers 421, 422, and 423. The plurality of transducers 421, 422, and 423 may be included in the rotating portion 313 of the base unit 310.

The plurality of transducers 421, 422, and 423 may be formed on one side of the base unit 310 and rotated together with the base unit 310. The plurality of transducers 421, 422, and 423 may vibrate at a predetermined therapeutic frequency to transmit vibration energy to the patient's skin.

At least one of the base unit 310 and the plurality of transducers 421, 422, and 423 may include a thermoelectric element (not shown). The thermoelectric element may be formed inside at least one of the base unit and the plurality of transducers 421, 422, and 423. The thermoelectric element may cool at least one of the base unit and the plurality of transducers to prevent burning of the skin surface of the patient in contact with one side of the handpiece.

The control unit 200 may determine the intensity of the vibration energy and emit the vibration energy from the plurality of transducers based on the intensity of the vibration energy. Since such a process has been described with reference to FIGS. 6, 7, and 9, the overlapping descriptions thereof will be omitted.

Referring to FIGS. 4, 5, and 9, the plurality of transducers 421, 422, and 423 may have predetermined different therapeutic frequencies. For example, the plurality of transducers 421, 422, and 423 may include a first transducer 421, a second transducer 422, and a third transducer 423. A predetermined therapeutic frequency of the first transducer 421 may be 1 MHz. A predetermined therapeutic frequency of the second transducer 422 may be 3 MHz. A predetermined therapeutic frequency of the third transducer 423 may be 10 MHz.

The plurality of transducers 421, 422, and 423 may emit vibration energy at the same time. For example, the plurality of transducers 421, 422, and 423 may emit vibration energy for a first time and stop emitting for a second time.

However, the present disclosure is not limited thereto, and the plurality of transducers 421, 422, and 423 may sequentially emit the vibration energy. For example, the first transducer 421 may operate for a third time and then stop, the second transducer 422 may operate for a fourth time and then stop, and the third transducer 423 may operate for a fifth time and then stop. In addition, the third time, the fourth time, and the fifth time may be repeated.

In addition, the plurality of transducers 421, 422, and 423 may sequentially emit the vibration energy only when located at specific locations with respect to the fixed portion 311. For example, referring to FIG. 4, an operating area 430 (dotted-line area) may be formed in the base unit 310. The plurality of transducers 421, 422, and 423 may emit the vibration energy only when located in the operating area 430 while rotating. In the case of FIG. 4, the first transducer 421 may operate. The operating area 430 may be changed based on the user's input. The plurality of transducers 421, 422, and 423 may determine whether to emit the vibration energy simultaneously, sequentially, or only when located in the operating area based on the user's input.

Referring to FIG. 4, a first angle 441 and a second angle 442 may be the same. The first angle may be an angle formed by a first line connecting a center of the first transducer 421 and a center (the rotational axis 410) of the base unit and a second line connecting a center of the second transducer 422 and the center (the rotational axis 410) of the base unit. The second angle 442 may be an angle formed by the first line connecting the center of the first transducer 421 and the center (the rotational axis 410) of the base unit and a third line connecting a center of the third transducer 423 and the center of the base unit.

A third angle may be the same as the first angle 441 and the second angle 442. The third angle may be an angle formed by the second line connecting the center of the second transducer 422 and the center (the rotational axis 410) of the base unit and a third line connecting a center of the third transducer 423 and the center (the rotational axis 410) of the base unit.

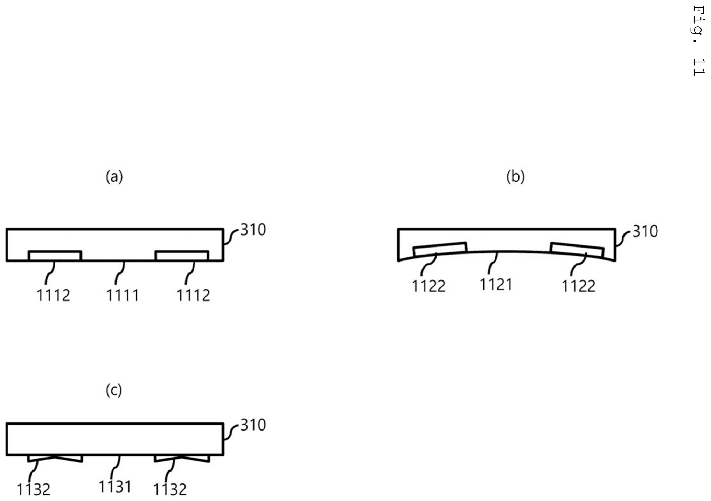

FIG. 11 is a view showing a cross section of a base unit according to one embodiment of the present disclosure.

FIG. 11 shows the rotating portion 313 of the base unit 310, with the fixed portion 311 omitted. Referring to FIG. 11A, one side of the base unit 310 may have a flat surface 1111. In addition, the plurality of transducers formed on the base unit 310 may have flat surfaces 1112. In this case, the plurality of transducers may treat different points below the patient's skin when stopped. However, the plurality of transducers may repeatedly treat the same area while rotating together with the base unit 310. When each of the plurality of transducers have the flat surface 1112, the plurality of transducers may treat an area as wide as the area of the plurality of transducers below the patient's skin.

Referring to FIG. 11B, the base unit 310 may have a concave surface 1121. The plurality of transducers may be formed on the concave surface 1121 of the base unit 310. The plurality of transducers 1122 may each have a flat surface. According to FIG. 11B, the plurality of transducers 1122 may treat almost the same point below the patient's skin even when stopped. However, the present disclosure is not limited thereto, and the plurality of transducers 1122 may treat different depths below the patient's skin. In the present disclosure, the term "concave" may mean that a surface is inclined downward from an edge to a center.

Referring to FIGS. 11A and 11C, the base unit 310 may have flat surfaces 1111 and 1131. In addition, the plurality of transducers may be formed on the flat surfaces 1111 and 1131 of the base unit 310. The plurality of transducers may have one of a concave surface 1132 and the flat surface 1112. When the plurality of transducers have the concave surfaces 1132, the plurality of transducers may treat different points below the patient's skin when stopped. However, the plurality of transducers may repeatedly treat the same area while rotating together with the base unit 310. In addition, when the plurality of transducers have the concave surfaces 1132, a focus is formed so that a specific point of the patient's skin may be intensively treated. In addition, the skin tissue may be destructed due to heat at the point at which the focus is formed. The destroyed skin tissue may naturally regenerate, thereby improving skin wrinkles. In addition, the destroyed skin tissue may be fat tissue, and the effect of reducing fat may be present as the fat tissue is destroyed.

Referring to FIG. 10, a suction hole for suctioning the patient's skin may be formed adjacent to the center (the rotational axis 410) of the circle of the base unit 310. The suction hole may be formed in the rotational axis 410. However, the present disclosure is not limited thereto, and the suction hole may be formed in at least one of a plurality of points 1011, 1012, and 1013. The plurality of points 1011, 1012, and 1013 may be formed between the plurality of transducers 421, 422, and 423.

At least one of the handpiece 130 or the main body 110 may include a negative pressure unit (not shown). The negative pressure unit may be a component configured to form a lower pressure than atmospheric pressure. For example, the negative pressure unit may be a compressor. When the negative pressure unit is formed in the main body 110, negative pressure may be transmitted to the suction holes 1011, 1012, and 1013 through a tube included in the cable 131. The handpiece 130 or the main body 110 may include a pressure sensor for measuring negative pressures formed in the suction holes 1011, 1012, and 1013.

The skin treatment device 100 may generate a lower pressure than the atmospheric pressure in the suction hole 1011, 1012, 1013, or 410 by the negative pressure unit. The suction hole 1011, 1012, 1013, or 410 may have a lower pressure than the atmospheric pressure to suction the patient's skin. Accordingly, the plurality of transducers 421, 422, and 423 formed in the base unit 310 may come into close contact with the patient's skin. The plurality of transducers 421, 422, and 423 may treat a predetermined location below the skin surface of the patient. To treat the predetermined location, the plurality of transducers 421, 422, and 423 need to be in contact with the skin. When the plurality of transducers 421, 422, and 423 are not in contact with the skin, the plurality of transducers 421, 422, and 423 may not transmit energy to the predetermined location. Based on the suction hole 1011, 1012, 1013, or 410, the plurality of transducers 421, 422, and 423 come into close contact with the skin, and thus may always treat the predetermined location. Accordingly, the treatment precision of the skin treatment device can be increased.

The control unit 200 may measure a distance between one side of the handpiece 130 and the patient's bone based on the plurality of transducers 421, 422, and 423 included in the handpiece 130. The plurality of transducers 421, 422, and 423 may generate ultrasound toward the patient's skin. The plurality of transducers 421, 422, and 423 may also receive ultrasound reflected from tissues under the patient's skin. The control unit 200 may measure the distance from the skin surface to the bone based on the reflected ultrasound. The control unit 200 may measure the distance from the skin surface to the bone based on the plurality of transducers 421, 422, and 423 before emitting therapeutic energy to the skin.

When the measured distance between the one side of the handpiece 130 and the patient's bone is within a threshold range and the pressure formed in the suction hole is less than a threshold pressure, the control unit 200 may control the plurality of transducers to emit the vibration energy. Here, the threshold range may be a first threshold distance or more and a second threshold distance or less. The handpiece 130 or the main body 110 may include a pressure sensor for measuring negative pressures formed in the suction holes 1011, 1012, and 1013. Based on the pressure sensor, the control unit 200 may measure the pressure formed in the suction holes 1011, 1012, and 1013.

In addition, when the measured distance is not included in the threshold range or the pressure formed in the suction hole is higher than or equal to the threshold pressure, the control unit 200 may control the plurality of transducers not to emit the vibration energy. In this way, by controlling the plurality of transducers to emit the vibration energy only when the measured distance between the one side and the patient's bone is included in the threshold range and the pressure formed in the suction hole is less than the threshold pressure, the patient's safety can be secured, and accurate treatment can be performed. This is because the skin treatment device can perform accurate treatment only when the distance between the one side of the handpiece 130 and the patient's bone is included in the threshold range. When the distance between the one side of the handpiece 130 and the patient's bone is outside the threshold range, it may damage nerves or muscles below the skin, thereby causing harm to the patient. In addition, when the pressure formed in the suction hole is less than the threshold pressure, it means that the suction hole holds the patient's skin, which may mean that it is ready to safely emit the therapeutic energy to the patient.

In addition, each of the plurality of transducers may measure the distance between the one side and the patient's bone. The control unit 200 may control only at least one of the plurality of transducers, in which the measured distance between the one side and the patient's bone is within the threshold range, to emit the vibration energy. Allowing only a ready transducer to emit the vibration energy may mean that the therapeutic energy is ready to be safely emitted to the patient.

The configuration in which the base unit 310 coupled to the handpiece 130 includes the plurality of transducers has been described above. Hereinafter, a configuration in which the base unit 310 coupled to the handpiece 130 includes at least one positive electrode will be described. When the base unit 310 coupled to the handpiece 130 includes at least one positive electrode, the therapeutic energy emitted by the handpiece 130 may be electrical energy.

Referring to FIG. 4, the base unit 310 may be formed on one side of the handpiece. At least one positive electrode (at least one of 421, 422, and 423) may be formed on one side of the base unit 310. The at least one positive electrode (at least one of 421, 422, and 423) may be a component for providing electrical energy to the patient's skin to improve the skin.

A plurality of positive electrodes are disclosed in FIG. 4, but the present disclosure is not limited thereto. The base unit 310 may include one positive electrode. In addition, the base unit 310 may include at least one positive electrode (at least one of 421, 422, and 423) and at least one negative electrode (at least one of 421, 422, and 423). That is, the at least one negative electrode (at least one of 421, 422, and 423) may be formed on the one side of the base unit included in the handpiece. In addition, when the at least one negative electrode is not formed on the one side of the base unit included in the handpiece, the skin treatment device 100 may include at least one negative electrode on a negative electrode pad independent of the handpiece.

The at least one positive electrode (at least one of 421, 422, and 423) and the at least one negative electrode (at least one of 421, 422, and 423) may have a circular shape. The at least one positive electrode (at least one of 421, 422, and 423) and at least one negative electrode (at least one of 421, 422, and 423) may have the same shape, but are not limited thereto. The at least one positive electrode (at least one of 421, 422, and 423) and the at least one negative electrode (at least one of 421, 422, and 423) may have a flat surface or a convex surface toward the patient's skin. When each of the at least one positive electrode (at least one of 421, 422, and 423) and the at least one negative electrode (at least one of 421, 422, and 423) have a convex surface, the electrodes can be in easy contact with the patient's skin. In addition, centers of the at least one positive electrode (at least one of 421, 422, and 423) and at least one negative electrode (at least one of 421, 422, and 423) may be spaced a predetermined distance from the rotational axis 410. The at least one positive electrode may rotate together with the base unit. Since the rotation has already been described, the detailed description thereof will be omitted.

According to various embodiments of the present disclosure, the handpiece 130 may not include a negative electrode. In this case, the skin treatment device 100 may have a separate negative electrode pad. A user may perform skin treatment using the handpiece 130 after bringing the negative electrode pad into contact with the patient.

According to various embodiments of the present disclosure, some of the reference numerals 421, 422, and 423 may be at least one negative electrode. For example, the handpiece 130 may include at least one positive electrode 421 and 422 and at least one negative electrode 423. In addition, at least one negative electrode may be formed on the rotational axis 410. That is, the handpiece 130 may have at least one positive electrode 421, 422, and 423 and at least one negative electrode on the rotational axis 410. However, the present disclosure is not limited thereto, and unlike FIG. 4, the at least one positive electrode 421, 422, and 423 may be disposed in a row. In this way, when the handpiece 130 has a negative electrode and a positive electrode, a separate negative electrode pad may not be required.

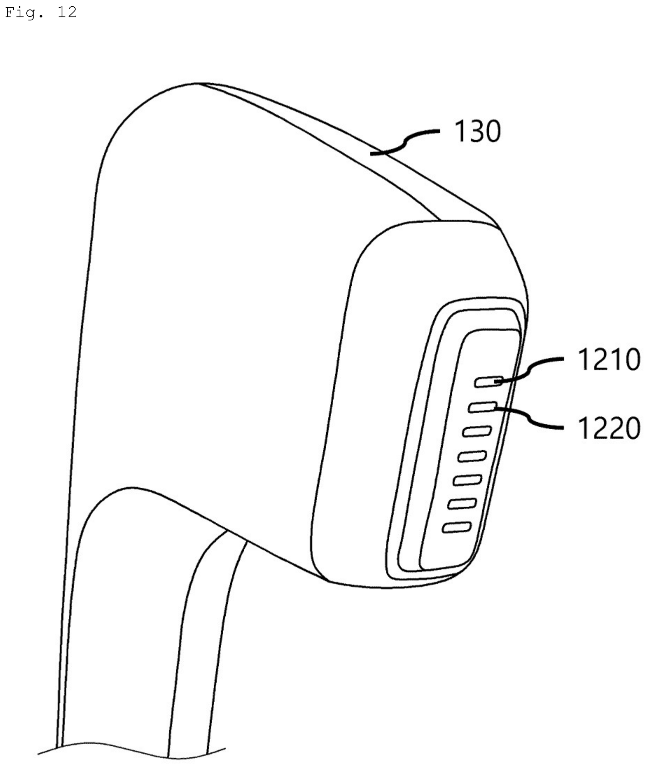

FIG. 12 is a view showing at least one positive electrode and at least one negative electrode according to one embodiment of the present disclosure.

Referring to FIG. 12, the handpiece 130 may include at least one positive electrode and at least one negative electrode. The at least one positive electrode and the at least one negative electrode may be disposed in a row. For example, an electrode adjacent to a positive electrode 1210 may be a negative electrode 1220.

Referring back to FIG. 4, at least one of the base unit 310 and the at least one positive electrode 421, 422, and 423 may include a thermoelectric element (not shown). The thermoelectric element may be formed inside at least one of the base unit 310 and the at least one positive electrode 421, 422, and 423. The thermoelectric element may cool at least one of the base unit and the plurality of transducers. Accordingly, the thermoelectric element can prevent burning of the skin surface of the patient in contact with one side of the handpiece 130.

Referring to FIGS. 4, 5, and 6, the control unit 200 may perform operation 610 of measuring the impedance of the patient's skin by allowing current to flow through the patient's skin or applying voltage to the patient's skin using at least one positive electrode and at least one negative electrode. The at least one positive electrode and the at least one negative electrode may be disposed on the reference numerals 421, 422, and 423. However, the present disclosure is not limited thereto, and the control unit 200 may perform operation 610 of measuring the impedance of the patient's skin by allowing current to flow through the patient's skin or applying voltage to the patient's skin using separate electrode units 511, 512, and 513.

The control unit 200 may perform operation 620 of determining the intensity of therapeutic energy corresponding to the impedance of the patient's skin based on a predetermined function. The therapeutic energy may include at least one of vibration energy and electrical energy. When the base unit 310 includes at least one of at least one positive electrode or at least one negative electrode, the therapeutic energy may be the electrical energy.

The control unit 200 may perform operation 630 of emitting the electrical energy from the at least one of the at least one positive electrode or the at least one negative electrode based on the intensity of the electrical energy. When the base unit 310 includes at least one of the at least one positive electrode or the at least one negative electrode, the therapeutic energy will be the electrical energy. The control unit 200 may control the therapeutic current to flow from the at least one positive electrode to the at least one negative electrode through the skin based on the intensity of the electrical energy and a predetermined therapeutic frequency. The therapeutic current may correspond to the therapeutic energy.

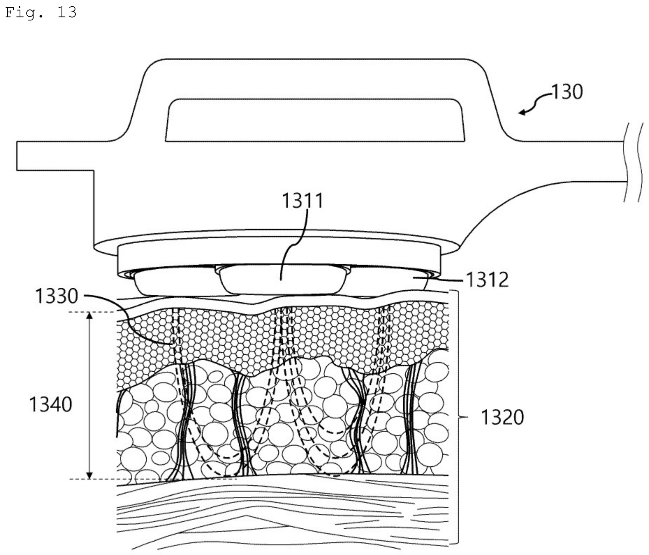

FIG. 13 is a view for describing the handpiece according to one embodiment of the present disclosure.

The handpiece 130 may include at least one positive electrode 1311 or at least one negative electrode 1312. As described above, the handpiece 130 may include only the positive electrode. In this case, the skin treatment device 100 may include a separate negative electrode pad.

The control unit 200 may control a therapeutic current 1330 to flow from the at least one positive electrode 1311 to the at least one negative electrode 1312 through a skin 1320 based on the intensity of the electrical energy and the predetermined therapeutic frequency. Here, the therapeutic current 1330 may correspond to the therapeutic energy.

A depth 1340 to which the electrical energy penetrates below the surface of the patient's skin 1320 via the handpiece 130 may be inversely proportional to an electrode distance between the at least one positive electrode 1311 and the at least one negative electrode 1312. Here, the depth 1340 to which the therapeutic energy penetrates may be a depth at which a lesion is formed. The therapeutic energy may be electrical energy. A lesion may be formed on a portion of the patient's skin by the electrical energy, and the patient's skin can be improved during the process of the lesion being recovered. In addition, the depth 1340 of forming a lesion below the surface of the patient's skin 1320 may be inversely proportional to the therapeutic frequency.

The therapeutic energy including the electrical energy may be provided to the patient in the form of a therapeutic waveform. The intensity (size) of the therapeutic energy may be determined by a peak value of the therapeutic waveform. The therapeutic waveform may have a therapeutic frequency. The therapeutic frequency may be 250 kHz or more and 20 MHz or less. The therapeutic waveform may be a sine wave. The intensity (size) of the therapeutic energy may be determined by the peak value of the therapeutic waveform, which is a sine wave. The therapeutic waveform may be in the form of a pulse. That is, the therapeutic waveform may be a form in which sine waves are provided for a predetermined first time, and the waves are not provided for a predetermined second time after the first time has elapsed. The first time and the second time may be repeated in succession.

Hereinafter, a process of measuring impedance by the handpiece 130 according to various embodiments of the present disclosure will be described with reference to FIGS. 7 and 13. Since FIG. 7 has already been described, the overlapping description thereof will be omitted.

Referring to FIGS. 7 and 13, the control unit 200 may perform operation 710 of controlling a predetermined test current to flow from the at least one positive electrode 1311 to the at least one negative electrode 1312. The handpiece 130 may measure impedance using the at least one positive electrode 1311 and the at least one negative electrode 1312 instead of the electrode unit 511, 512, and 513 described above. The control unit 200 may perform operation 720 of measuring a test voltage between the at least one positive electrode 1311 and the at least one negative electrode 1312. The control unit 200 may perform operation 730 of measuring the impedance of the patient's skin based on the test current and the test voltage.

In addition, the control unit 200 may perform operation 740 of determining the intensity of the electrical energy to be increased as the impedance increases based on a predetermined function. The predetermined function may have the form of a logarithmic function. That is, in a coordinate system in which a horizontal axis represents impedance and a vertical axis represents an intensity of electrical energy, the predetermined function may be represented by a logarithmic function. Since the logarithmic function has already been described with reference to FIG. 8, the detailed description thereof will be omitted.

The intensity of the electrical energy may be determined based on at least one of a level of a voltage or an amount of a current. The high intensity of the electrical energy may mean that the voltage is high or the current is high. The control unit 200 may perform operation 750 of emitting the electrical energy from at least one positive electrode to at least one negative electrode based on the intensity of the electrical energy.

Hereinafter, a process of measuring impedance by the handpiece 130 according to various embodiments of the present disclosure will be described with reference to FIGS. 9 and 13. Since FIG. 9 has already been described, the overlapping description thereof will be omitted.

The control unit 200 may perform operation 710 of controlling the electrode unit to apply the test voltage between at least one positive electrode and at least one negative electrode. The control unit 200 may perform operation 720 of measuring the test current flowing between at least one positive electrode and at least one negative electrode. The control unit 200 may perform operation 730 of measuring the impedance of the patient's skin based on the test current and the test voltage. The control unit 200 may perform operation 740 of determining the intensity of the electrical energy to be increased as the impedance increases based on the predetermined function. The intensity of the electrical energy may be determined based on at least one of a level of voltage or an amount of current. The high intensity of the electrical energy may mean that the level of voltage or the amount of current is increased. The control unit 200 may perform operation 750 of emitting the electrical energy from at least one positive electrode to at least one negative electrode based on the intensity of the electrical energy.

Referring to FIG. 10, the base unit 310 may include the suction hole 1011, 1012, 1013, or 410 for suctioning the patient's skin. At least one of the handpiece 130 or the main body 110 may include a negative pressure unit (not shown). The negative pressure unit may be a component configured to form a lower pressure than atmospheric pressure. The skin treatment device 100 may generate a lower pressure than the atmospheric pressure in the suction hole 1011, 1012, 1013, or 410 by the negative pressure unit. The suction hole 1011, 1012, 1013, or 410 may have a lower pressure than the atmospheric pressure to suction the patient's skin.

The control unit 200 may determine the level of the pressure formed in the suction hole based on the measured impedance. For example, the control unit 200 may increase the level of the pressure when the impedance of the patient's skin is low. As described above, the low impedance of the patient's skin may mean a high moisture content and mean that the skin is elastic. In this case, even when the pressure formed in the suction hole is slightly lower than the atmospheric pressure, the skin may come into firm contact with the one side of the handpiece 130. However, high impedance may mean a lack of moisture, and when there is a lack of moisture, the skin may not come into firm contact with the one side of the handpiece 130 due to wrinkles. Accordingly, the pressure formed in the suction hole may be largely reduced so that the patient's skin is suctioned with a large force and comes into firm contact with the one side of the handpiece 130. However, the present disclosure is not limited thereto. The control unit 200 may also lower the level of the pressure when the impedance of the patient's skin is low.

The control unit 200 may measure the distance between the one side of the handpiece and the patient's bone based on the sensor included in the handpiece. The distance between the one side of the handpiece 130 and the patient's bone may be a distance from the skin surface to the bone. The sensor may be at least one transducer. In FIG. 5, the base unit 310 may include at least one positive electrode 421, 422, or 423 and at least one negative electrode 421, 422, or 423. In addition, the base unit 310 may include at least one transducer (at least one of 511, 512, and 513). The at least one transducer (at least one of 511, 512, and 513) may generate ultrasound toward the patient's skin. The at least one transducer (at least one of 511, 512, and 513) may receive ultrasound reflected from tissue below the patient's skin. The control unit 200 may measure the distance from the skin surface to the bone based on the reflected ultrasound. The control unit 200 may measure the distance from the skin surface to the bone based on at least one transducer (at least one of 511, 512, and 513) before emitting therapeutic energy to the skin.

When the measured distance is within the threshold range and the pressure formed in the hole is lower than or equal to the threshold pressure, the control unit 200 may control the at least one transducer to emit electrical energy of a predetermined therapeutic frequency to the patient's skin. In addition, when the measured distance is not included in the threshold range or the pressure formed in the suction hole is higher than or equal to the threshold pressure, the control unit 200 may control the at least one transducer not to emit the electrical energy. In this way, by controlling the at least one transducer to emit the electrical energy only when the measured distance between the one side and the patient's bone is included in the threshold range and the pressure formed in the suction hole is less than the threshold pressure, the patient's safety can be secured, and accurate treatment can be performed. This is because the skin treatment device can perform accurate treatment only when the distance between one side of the handpiece 130 and the patient's bone is included in the threshold range. When the distance between the one side of the handpiece 130 and the patient's bone is outside the threshold range, it may damage nerves or muscles below the skin, thereby causing harm to the patient. In addition, when the pressure formed in the suction hole is less than the threshold pressure, it means that the suction hole holds the patient's skin, which may mean that it is ready to safely emit the therapeutic energy to the patient.

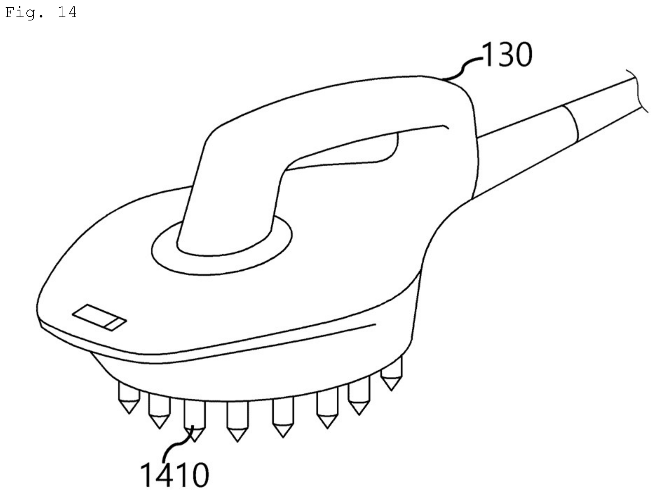

FIG. 14 is a view showing the handpiece according to one embodiment of the present disclosure.

The skin treatment device 100 may include the handpiece 130. The handpiece 130 may receive power from the power supply of the main body 110, may be controlled by the control unit 200, and may inject a drug into the patient's skin based on a protrusion 1410 formed on one side, thereby improving the skin.

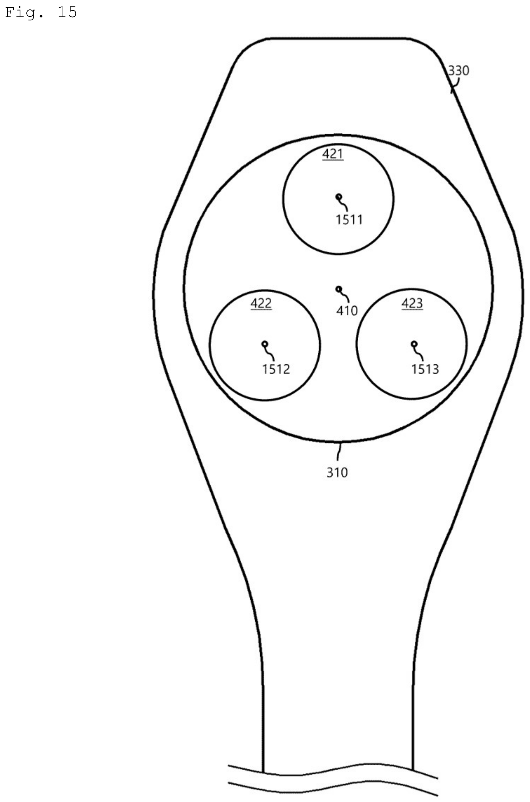

As described above, various types of base units 310 may be coupled to the handpiece 130. One of the various types of base units 310 may inject a drug into the patient's skin. At least one protrusion 1410 may be formed on the base unit 310. The at least one protrusion 1410 may provide the drug to the patient's skin without passing through the patient's skin. That is, the handpiece 130 may inject the drug into the patient's skin without a needle. A structure of the handpiece will be described in more detail with reference to FIG. 15.

FIG. 15 is a view showing one side of the handpiece according to one embodiment of the present disclosure.

The handpiece 130 may include the base unit 310 coupled to one side of the handpiece 130. The handpiece 130 may include the drug injection unit (at least one of 421, 422, and 423) that is formed on one side of the base unit 310 and injects a drug into the patient's skin. FIG. 15 shows three drug injection units, but the present disclosure is not limited thereto, and more drug injection units may be formed on the base unit 310.

The base unit 310 may include at least one drug discharge port (at least one of 1511, 1512, and 1513). In addition, the drug injection unit (at least one of 421, 422, and 423) may include at least one drug discharge port (at least one of 1511, 1512, and 1513). FIG. 15 shows one drug discharge port in one drug injection unit, but the present disclosure is not limited thereto, and the one drug injection unit may include at least one drug discharge port. The drug discharge port (at least one of 1511, 1512, and 1513) may correspond to a drug discharge port 1621.

Although not disclosed in FIG. 15, the base unit 310 may include at least one of a plurality of transducers, at least one positive electrode, at least one negative electrode, and an electrode unit in addition to the drug injection unit (at least one of 421, 422, and 423). More specifically, the base unit 310 may include at least one of a plurality of transducers that vibrate at a predetermined therapeutic frequency to transmit vibration energy to the patient's skin and at least one positive electrode for providing electrical energy to the patient's skin, thereby improving the skin. In addition, a drug injection port may be formed inside the plurality of transducers and at least one positive electrode. That is, the drug injection unit may be included inside at least one of the plurality of transducers and the at least one positive electrode. For example, in FIG. 15, the drug injection unit may be included inside the plurality of transducers (at least one of 421, 422, and 423), and a hole formed in the centers of the plurality of transducers (at least one of 421, 422, and 423) may be the drug discharge port (at least one of 1511, 1512, and 1513). In this way, since the base unit may play multiple roles, the burden of the user having to change the base unit can be reduced.

Since at least one of the plurality of transducers, the at least one positive electrode, the at least one negative electrode, the electrode unit, and the suction hole has been described with reference to FIGS. 4, 5, and 10, the overlapping descriptions thereof will be omitted.

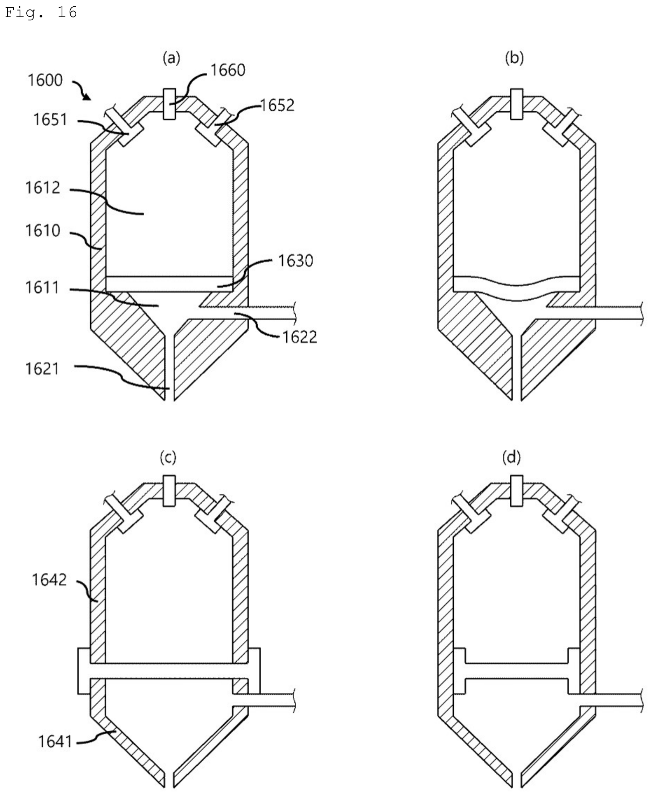

Hereinafter, the drug injection unit (at least one of 421, 422, and 423) will be described with reference to FIG. 16.

FIG. 16 is a view showing a cross section of a drug injection unit according to one embodiment of the present disclosure.

A drug injection unit 1600 may include a drug cylinder 1610 including a first chamber 1611 and a second chamber 1612. The first chamber 1611 may receive and store a drug from a drug supply unit. The second chamber 1612 may accommodate an expansion fluid for discharging the drug of the first chamber 1611.

The drug injection unit 1600 may include a separator 1630. The separator may be formed between the first chamber 1611 and the second chamber 1612. The separator 1630 can prevent a fluid from flowing between the first chamber 1611 and the second chamber 1612. In addition, the separator 1630 may be made of an elastic material.

Referring to FIGS. 16A, 16B, and 16C, the drug cylinder 1610 may be formed integrally. The separator 1630 may be attached to the inside of the drug cylinder 1610 to separate the first chamber 1611 and the second chamber 1612. The separator 1630 may be adhered to the inside of the drug cylinder 1610 using an adhesive. Due to the separator 1630, the fluid in the first chamber 1611 may not flow into the second chamber 1612, and the fluid in the second chamber 1612 may not flow into the first chamber 1611.

Referring to FIG. 16C, the drug cylinder 1610 may include a first drug cylinder 1641 and a second drug cylinder 1642. The first drug cylinder 1641 may form the first chamber 1611, and the second drug cylinder 1642 may form the second chamber 1612. The first drug cylinder 1641 and the second drug cylinder 1642 may be adhered by the separator 1630. The drug cylinder 1610 such as FIG. 16C has an advantage of being easy to assemble.

The drug injection unit 1600 may include an injection valve 1651. The injection valve 1651 may be formed in the second chamber 1612. The injection valve 1651 may be formed on an upper end of the drug cylinder 1610. However, the present disclosure is not limited thereto. The injection valve 1651 may be a component for injecting an expansion fluid of a predetermined mass into the second chamber 1612.

The drug injection unit 1600 may include an ignition plug 1660. The ignition plug 1660 may be formed in the second chamber 1612. The ignition plug 1660 may be formed on the upper end of the drug cylinder 1610. The ignition plug 1660 may be a component for expanding the expansion fluid injected into the second chamber 1612.

The drug injection unit 1600 may include a discharge valve 1652. The discharge valve 1652 may be formed in the second chamber 1612. The discharge valve 1652 may be formed on the upper end of the drug cylinder 1610. However, the present disclosure is not limited thereto. The discharge valve 1652 may be a component for discharging the expansion fluid expanded by the ignition plug 1660 of the second chamber 1612 from the second chamber 1612.

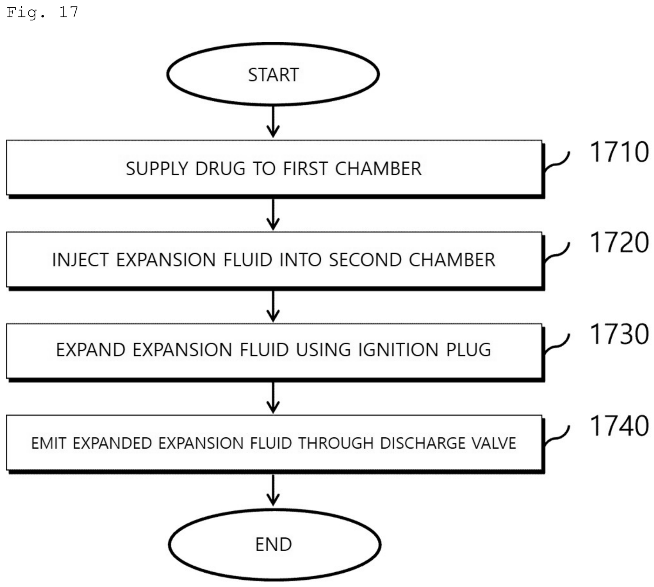

FIG. 17 is a flowchart showing operations of the skin treatment device according to one embodiment of the present disclosure.

The control unit 200 may perform operation 1710 of supplying a drug from a drug supply unit (not shown) to the first chamber 1611. The drug supply unit may be included in at least one of the base unit 310 and the main body 330 of the handpiece 130. The drug supply unit may be preferably included in the base unit 310. The user may select the base unit 310 including a drug suitable for treating the patient and couple the base unit 310 to the main body 330 of the handpiece 130. In addition, the patient may be treated using the handpiece 130 to which the base unit 310 is coupled. The first chamber 1611 may receive a drug through a drug injection port 1622. The drug injection port 1622 may be formed on a side surface of the drug cylinder 1610.

The medical skin treatment device 100 may include a pressure sensor for measuring a pressure of the first chamber 1611. The pressure sensor may be included in at least one of the drug injection port 1622, the drug supply unit, and the first chamber 1611.

The control unit 200 may measure the pressure of the first chamber 1611 when supplying the drug from the drug supply unit to the first chamber 1611. In addition, the control unit 200 may stop supplying the drug to the first chamber when the measured pressure of the first chamber 1611 is higher than or equal to the threshold pressure. The first chamber 1611 needs to store an appropriate amount of the drug. When the first chamber 1611 stores too much drug, the separator 1630 may be convex toward the second chamber 1612, thereby reducing the durability of the separator 1630. In addition, when the first chamber 1611 stores too much drug, there is a possibility that a drug having an amount more than the appropriate amount may be injected into the patient. Accordingly, the skin treatment device of the present disclosure may supply an appropriate amount of drug to the first chamber 1611 using the pressure sensor.

The control unit 200 may perform operation 1720 of opening the injection valve 1651 to inject an expansion fluid into the second chamber 1612. The expansion fluid may be supplied from an expansion fluid storage unit (not shown) of the base unit 310. The user may fill the base unit 310 with the expansion fluid. The injection valve 1651 may supply a predetermined amount of expansion fluid to the second chamber 1612.

The control unit 200 may perform operation 1730 of expanding the expansion fluid using the ignition plug 1660 after closing the injection valve 1651. The expansion fluid may be, for example, gas or a liquid that expands by a spark. The ignition plug 1660 may provide energy for the expansion fluid to expand. The energy for expansion may be a spark by electricity.

The first chamber 1611 may include the drug discharge port 1621 for discharging a drug and the drug injection port 1622 for supplying a drug from the drug supply unit at a lower side thereof. The drug cylinder 1610 may include at least one drug discharge port (at least one of 1511, 1512, and 1513). The separator 1630 may be convex toward the first chamber 1611 due to the expansion of the expansion fluid. That is, the separator 1630 may apply pressure to the drug inside the first chamber 1611 so that the drug may be discharged through the drug discharge port 1621. Accordingly, the drug in the first chamber 1611 may be injected into the patient's skin through the drug discharge port 1621. The drug discharge port (at least one of 1511, 1512, and 1513) may correspond to the drug discharge port 1621.

The first chamber 1611 may have a shape of which an inner diameter decreases in a downward direction or is constant. An outer diameter of the first chamber 1611 may decrease or may not decrease in the downward direction. The first chamber 1611 may include the drug discharge port 1621 on a lower end thereof. The drug discharge port 1621 may have a very small inner diameter compared to that of the first chamber 1611. For example, an inner diameter of the drug discharge port 1621 may be 10 micrometers or more and 300 micrometers or less. Since the inner diameter of the drug discharge port 1621 is small, the drug from the drug discharge port 1621 may be discharged at a very high pressure. Accordingly, the drug injection unit 1600 may inject the drug into the patient's skin without a needle.

The control unit 200 may perform operation 1740 of opening the discharge valve 1652 so that the expanded expansion fluid is discharged from the second chamber 1612 through the discharge valve 1652. The control unit 200 may simultaneously perform operation 1740 of opening the discharge valve 1652 so that the expanded expansion fluid is discharged from the second chamber 1612 through the discharge valve 1652 and operation 1710 of supplying the drug to the first chamber 1611. Accordingly, the expanded expansion fluid in the second chamber 1612 may be discharged through the discharge valve 1652 by the pressure of supplying the drug to the first chamber 1611. However, the present disclosure is not limited thereto, and operation 1710 may be performed after operation 1740. The expanded expansion fluid may be discharged from the second chamber 1612 through the discharge valve by an elastic force of the separator 1630. That is, the expanded expansion fluid in the second chamber 1612 may be discharged through the discharge valve 1652 by a restoring force of the separator 1630. In addition, the control unit 200 may repeatedly perform operations 1710 to 1740.

The configuration using the ignition plug 1660 has been mainly described above, but the present disclosure is not limited thereto. The drug injection unit 1600 may include only the injection valve 1651 and the discharge valve 1652 instead of the injection valve 1651, the discharge valve 1652, and the ignition plug 1660. The control unit 200 may perform operation 1710 of supplying a drug from a drug supply unit (not shown) to the first chamber 1611. The control unit 200 may perform operation 1720 of opening the injection valve 1651 to inject an expansion fluid into the second chamber 1612. Here, the expansion fluid may be general air. The injection valve 1651 may supply the expansion fluid at a pressure much higher than the pressure of the first chamber 1611. For example, the supply pressure of the expansion fluid may be 10 times higher than the pressure inside the first chamber 1611. The injection valve 1651 may supply a predetermined mass of the expansion fluid. The separator 1630 may be convex toward the first chamber 1611 due to the expansion fluid supplied to the second chamber 1612. That is, the separator 1630 may apply pressure to the drug in the first chamber 1611. Accordingly, the drug in the first chamber 1611 may be injected into the patient's skin through the drug discharge port 1621. The control unit 200 may perform operation 1740 of opening the discharge valve 1652 so that the expansion fluid is discharged from the second chamber 1612 through the discharge valve 1652. The expansion fluid may be discharged from the second chamber 1621 through the discharge valve by the elastic force of the separator 1630. That is, the expansion fluid in the second chamber 1612 may be discharged through the discharge valve 1652 by the restoring force of the separator 1630. Operation 1710 may be performed after operation 1740. In addition, the control unit 200 may repeatedly perform operations 1710 to 1740.

Various embodiments have been mainly described so far. Those skilled in the art to which the present disclosure pertains will understand that the present disclosure may be implemented in a modified form without departing from the essential characteristics of the present disclosure. Accordingly, the disclosed embodiments should be considered in an illustrative rather than a limiting sense. The scope of the present invention is described in the claims rather than the above description, and all differences in the equivalent scope should be construed as being included in the present invention.

Accordingly, the above embodiments of the present disclosure may be prepared as a computer-executable program and implemented in a general-purpose digital computer for operating the program using a computer-readable storage medium. The computer-readable recording medium may include a storage medium such as a magnetic storage medium (e.g., a read only memory (ROM), a floppy disk, a hard disk, etc.) or an optical reading medium (e.g., a compact disc-ROM (CD-ROM), a digital video disc (DVD), etc.).

**Claims**

1.  A medical skin treatment device comprising:

    a main body including a control unit configured to control an operation of the skin treatment device and a power source unit configured to supply power to the medical skin treatment device;
    a transfer unit configured to move the main body; and
    a handpiece that receives power from the power source unit of the main body, is controlled by the control unit, and has one side that comes into contact with a patient's skin to form a lesion based on vibration energy below a skin surface of the patient so as to improve skin,
    wherein the handpiece includes:

    a base unit coupled to the one side of the handpiece;
    a plurality of transducers that are formed on one side of the base unit at positions excluding a center of the base unit and vibrate at a predetermined therapeutic frequency to transmit the vibration energy to the patient's skin;
a thermoelectric element that is formed inside at least one of the base unit and the plurality of transducers and cools the at least one of the base unit and the plurality of transducers to prevent burning of the skin surface of the patient in contact with the one side of the handpiece; and
an electrode unit that is formed on the one side of the base unit, includes at least one positive electrode and at least one negative electrode, and measures an impedance of the patient's skin, and
the control unit determines an intensity of the vibration energy corresponding to the impedance of the patient's skin based on a predetermined function and emits the vibration energy from the plurality of transducers based on the intensity of the vibration energy.

2.  The medical skin treatment device of claim 1, wherein, in a coordinate system in which a horizontal axis represents the impedance and a vertical axis represents the intensity of the vibration energy, the predetermined function is represented by a logarithmic function.

3.  The medical skin treatment device of claim 2, wherein the control unit is configured to:

    cause the electrode unit to flow a predetermined test current from the at least one positive electrode to the at least one negative electrode;
    measure a test voltage between the at least one positive electrode and the at least one negative electrode;
    measure the impedance of the patient's skin based on the test current and the test voltage;
    determine the intensity of the vibration energy to be increased as the impedance increases based on the predetermined function; and
    control at least one of the plurality of transducers to emit the vibration energy based on the intensity of the vibration energy.

4.  The medical skin treatment device of claim 2, wherein the control unit is configured to:

    control the electrode unit to apply a test voltage between the at least one positive electrode and the at least one negative electrode;
    measure a test current flowing between the at least one positive electrode and the at least one negative electrode;
    measure the impedance of the patient's skin based on the test current and the test voltage;
    determine the intensity of the vibration energy to be increased as the impedance increases based on the predetermined function; and control at least one of the plurality of transducers to emit the vibration energy based on the intensity of the vibration energy.

5. The medical skin treatment device of claim 2, wherein the predetermined therapeutic frequency of the vibration energy is 250 kHz or more and 20 MHz or less.

6. The medical skin treatment device of claim 5, wherein a suction hole for suctioning the patient's skin is formed in the base unit, and

   a negative pressure unit included in at least one of the handpiece or the main body forms a lower pressure than atmospheric pressure in the suction hole to suction the patient's skin.

7. The medical skin treatment device of claim 6, wherein a distance between the one side of the handpiece and the patient's bone is measured based on the plurality of transducers included in the handpiece,

   when the measured distance is included in a threshold range and the pressure formed in the suction hole is less than a threshold pressure, the control unit controls the plurality of transducers to emit the vibration energy, and
   when the measured distance is not included in the threshold range or the pressure formed in the suction hole is higher than or equal to the threshold pressure, the control unit controls the plurality of transducers not to emit the vibration energy.

8. The medical skin treatment device of claim 7, wherein at least one of the threshold range and the threshold pressure is determined based on the measured impedance.

Fig. 1

Fig. 2

EP 4 643 943 A1

100

200

CONTROL UNIT

SENSOR UNIT — 210

COMMUNICATION UNIT — 220

MEMORY — 230

OUTPUT UNIT — 240

INPUT UNIT — 250

EP 4 643 943 A1

Fig. 3

(a)

130

(b)

130

Fig. 4

Fig. 5

Fig. 6

```
START
  │
  ▼
MEASURE IMPEDANCE OF SKIN                              610
  │
  ▼
DETERMINE INTENSITY OF THERAPEUTIC ENERGY BASED       620
ON IMPEDANCE OF SKIN
  │
  ▼
EMIT THERAPEUTIC ENERGY TO SKIN                       630
  │
  ▼
END
```

Fig. 7

```
                    ┌─────────────┐
                    │    START    │
                    └──────┬──────┘
                           │
                           ▼
┌──────────────────────────────────────────────┐
│         CONTROL TEST CURRENT TO FLOW          │  ⟿ 710
└──────────────────────┬───────────────────────┘
                       │
                       ▼
┌──────────────────────────────────────────────┐
│              MEASURE TEST VOLTAGE             │  ⟿ 720
└──────────────────────┬───────────────────────┘
                       │
                       ▼
┌──────────────────────────────────────────────┐
│              MEASURE IMPEDANCE                │  ⟿ 730
└──────────────────────┬───────────────────────┘
                       │
                       ▼
┌──────────────────────────────────────────────┐
│          DETERMINE INTENSITY OF ENERGY        │  ⟿ 740
└──────────────────────┬───────────────────────┘
                       │
                       ▼
┌──────────────────────────────────────────────┐
│                 EMIT ENERGY                   │  ⟿ 750
└──────────────────────┬───────────────────────┘
                       │
                       ▼
                 ┌───────────┐
                 │    END    │
                 └───────────┘
```

Fig. 8

Fig. 9

```
          ┌─────────────┐
          │    START    │
          └──────┬──────┘
                 │
                 ▼
   ┌──────────────────────────────────┐      910
   │  CONTROL TEST VOLTAGE TO BE APPLIED │
   └─────────────────┬────────────────┘
                     │
                     ▼
   ┌──────────────────────────────────┐      920
   │       MEASURE TEST CURRENT        │
   └─────────────────┬────────────────┘
                     │
                     ▼
   ┌──────────────────────────────────┐      730
   │        MEASURE IMPEDANCE          │
   └─────────────────┬────────────────┘
                     │
                     ▼
   ┌──────────────────────────────────┐      740
   │    DETERMINE INTENSITY OF ENERGY  │
   └─────────────────┬────────────────┘
                     │
                     ▼
   ┌──────────────────────────────────┐      750
   │           EMIT ENERGY             │
   └─────────────────┬────────────────┘
                     │
                     ▼
          ┌─────────────┐
          │     END     │
          └─────────────┘
```

Fig. 10

Fig. 11

(a)

(b)

(c)

310

1112 1111 1112

1122 1121 1122

1132 1131 1132

Fig. 12

130

1210

1220

Fig. 13

Fig. 14

Fig. 15

Fig. 16

Fig. 17

```
                    ┌─────────────┐
                    │    START    │
                    └──────┬──────┘
                           │
                           ▼
    ┌──────────────────────────────────────────┐
    │     SUPPLY DRUG TO FIRST CHAMBER          │──── 1710
    └──────────────────────┬───────────────────┘
                           │
                           ▼
    ┌──────────────────────────────────────────┐
    │   INJECT EXPANSION FLUID INTO SECOND CHAMBER │──── 1720
    └──────────────────────┬───────────────────┘
                           │
                           ▼
    ┌──────────────────────────────────────────┐
    │   EXPAND EXPANSION FLUID USING IGNITION PLUG │──── 1730
    └──────────────────────┬───────────────────┘
                           │
                           ▼
    ┌──────────────────────────────────────────┐
    │  EMIT EXPANDED EXPANSION FLUID THROUGH DISCHARGE VALVE │──── 1740
    └──────────────────────┬───────────────────┘
                           │
                           ▼
                    ┌─────────────┐
                    │     END     │
                    └─────────────┘
```

## INTERNATIONAL SEARCH REPORT

| International application No. |
|---|
| **PCT/KR2022/021656** |

| A. | CLASSIFICATION OF SUBJECT MATTER |
|---|---|

**A61N 7/00**(2006.01)i; **A61B 5/0531**(2021.01)i

According to International Patent Classification (IPC) or to both national classification and IPC

| B. | FIELDS SEARCHED |
|---|---|

Minimum documentation searched (classification system followed by classification symbols)

A61N 7/00(2006.01); A61B 17/00(2006.01); A61B 17/50(2006.01); A61B 18/20(2006.01); A61B 5/00(2006.01); A61B 5/053(2006.01); A61N 1/04(2006.01); A61N 1/18(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 피부치료(skin treatment), 임피던스 측정(impedance measure), 진동 에너지 (vibration energy), 흡인홀(suction hole), 거리(distance), 압력(pressure)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2012-0090007 A (PRIMAEVA MEDICAL, INC.) 16 August 2012 (2012-08-16) See claims 25-73; and paragraph [0126]. | 1-6 |
| A |  | 7-8 |
| Y | KR 10-2366559 B1 (MEDICON CO., LTD.) 23 February 2022 (2022-02-23) See claim 10; and paragraph [0109]. | 1-6 |
| Y | KR 10-2017-0068697 A (HIRONIC CO., LTD.) 20 June 2017 (2017-06-20) See claim 2. | 6 |
| A | KR 10-2022-0143283 A (KIM, Yong Ho) 25 October 2022 (2022-10-25) See entire document. | 1-8 |
| A | KR 10-2016-0098480 A (L'OREAL) 18 August 2016 (2016-08-18) See entire document. | 1-8 |

| ☐ Further documents are listed in the continuation of Box C. | ☑ See patent family annex. |
|---|---|

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 September 2023** | **18 September 2023** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2022/021656**

| Patent document cited in search report | | | Publication date (day/month/year) | Patent family member(s) | | | Publication date (day/month/year) |
|---|---|---|---|---|---|---|---|
| KR | 10-2012-0090007 | A | 16 August 2012 | EP | 2401025 | A1 | 04 January 2012 |
| | | | | EP | 2401025 | A4 | 10 April 2013 |
| | | | | EP | 2401025 | B1 | 28 September 2016 |
| | | | | WO | 2010-098784 | A1 | 02 September 2010 |
| KR | 10-2366559 | B1 | 23 February 2022 | KR | 10-2021-0127606 | A | 22 October 2021 |
| KR | 10-2017-0068697 | A | 20 June 2017 | WO | 2017-099386 | A1 | 15 June 2017 |
| KR | 10-2022-0143283 | A | 25 October 2022 | | None | | |
| KR | 10-2016-0098480 | A | 18 August 2016 | EP | 3082576 | A2 | 26 October 2016 |
| | | | | EP | 3082576 | B1 | 11 March 2020 |
| | | | | JP | 2017-503568 | A | 02 February 2017 |
| | | | | US | 2015-0173996 | A1 | 25 June 2015 |
| | | | | WO | 2015-092587 | A2 | 25 June 2015 |
| | | | | WO | 2015-092587 | A3 | 29 October 2015 |

Form PCT/ISA/210 (patent family annex) (July 2022)

**EP 4 643 943 A1**

**Patent documents cited in the description**

- KR 20160000143